# EUROPEAN PATENT SPECIFICATION

(11) **EP 1 697 530 B1**
(45) Date of publication and mention of the grant of the patent: **31.03.2010**
(21) Application number: 04803729.5
(22) Date of filing: 10.12.2004
(51) Int. Cl.: C12P 13/08, C12N 15/31, C07K 14/245, C07K 14/25, C07K 14/225

(54) **PROCESS FOR PREPARING L-AMINO ACIDS USING STRAINS OF THE ENTEROBACTERIACEAE FAMILY**
VERFAHREN ZUR HERSTELLUNG VON L-AMINOSÄUREN UNTER VERWENDUNG VON STÄMMEN DER FAMILIE ENTEROBACTERIACEAE
PROCEDE DE FABRICATION DE L-ACIDES AMINES AU MOYEN DE SOUCHES DE LA FAMILLE DES ENTEROBACTERIACEES

(30) Priority: 24.12.2003 DE 10361268
(43) Date of publication of application: 06.09.2006
(73) Proprietor: Evonik Degussa GmbH, 45128 Essen (DE)
(72) Inventor: DUSCH, Nicole, 33824 Werther (DE)
(86) International application number: PCT/EP2004/014082
(87) International publication number: WO 2005/064000

(56) References cited:
- WO-A-03/008600
- WO-A-03/076637
- DATABASE UNI-PROT YAAU_ECOLI 1 November 1997 (1997-11-01), XP002321166 Database accession no. P31679
- DATABASE UNI-PROT Q83SQ5_SHIFL 1 June 2003 (2003-06-01), XP002321167 Database accession no. Q83SQ5
- DATABASE UNI-PROT Q8ZRW7_SALTY 1 October 2003 (2003-10-01), XP002321168 Database accession no. Q8ZRW7

## Description

This invention relates to a process for preparing L-lysine, L-valine and L-threonine, using recombinant microorganisms strains of the Enterobacteriaceae family in which the open reading frame (ORF) designated yaaU is overexpressed, and to said microorganisms.

### Prior art

L-Amino acids, in particular L-threonine, are used in human medicine and in the pharmaceutical industry, in the foodstuff industry and, very particularly, in animal nutrition.

It is known that L-amino acids can be prepared by fermenting Enterobacteriaceae strains, in particular Escherichia coli (E. coli) and Serratia marcescens. Because of the great importance, efforts are continually being made to improve the preparation methods. Methodological improvements can concern measures relating to fermentation technology, such as stirring or supplying with oxygen, or the composition of the nutrient media, such as the sugar concentration during the fermentation, or the working-up to the product form, for example by means of ion exchange chromatography, or the intrinsic performance properties of the microorganism itself.

Methods of mutagenesis, selection and mutant choice are used for improving the performance properties of these microorganisms. This thereby results in strains which are resistant to antimetabolites, such as the threonine analog α-amino-β-hydroxyvaleric acid (AHV), or auxotrophic for metabolites of regulatory importance and produce L-amino acids such as L-threonine.

For a number of years now, recombinant DNA methods have also been used for improving L-amino acid-producing strains of the Enterobacteriaceae family by amplifying individual amino acid biosynthesis genes and investigating the effect on production. Compiled information on the cell biology and molecular biology of Escherichia coli and Salmonella can be found in Neidhardt (ed): Escherichia coli and Salmonella, Cellular and Molecular Biology, 2nd edition, ASM Press, Washington, D.C., USA, (1996).

Documents D1-D3 disclose the amino acid sequences of the polypeptides as in SEQ ID NO: 4, 6 and 8 of present application, respectively.
- D1: DATABASE UNI-PROT YAAU_ECOLI, 1 November 1997 (1997-11-01), XP 002321166 Database accession no. P31679
abstract
encompasses a hypothetical metabolite tran sport protein yaaU from Escherichia coli
- D2: DATABASE UNI-PROT Q83Q5_SHIFL 1 June 2003 (2003-06-01), XP002321167 Database accession No. Q83SQ5
abstract
comprises a putative transport protein from Shigella flexneri
- D3: DATABASE UNI-PROT Q8ZRW7_SALTY 1 October 2003 (2003-10-01), XP002321168 Database accession no. Q8ZRW7
abstract
comprises a putative MFS family transport protein from Salmonella typhimurium.

Documents D4 WO 03/076637A and (D5) WO 03/008600A disclose methods for the production of L-threonine by means of overexpression or deletion of certain genes.

The problem to be solved in (D4) is regarded as the provision of a method for preparation of L-threonine by fermentating microorganism of the Enterobacteriaceae family which comprise an overexpressed pepB gene.

### Object of the invention

The object of this invention is to provide novel measures for improving the preparation of L-amino acids, in particular L-threonine.

### Description of the invention

The invention relates to recombinant microorganisms of the Enterobacteriaceae family which contain an overexpressed yaaU-ORF, which encodes a polypeptide which is annotated as being a putative sugar transporter, and which produce L-lysine, L-valine or L-threonine, in an improved manner.

In each case, the microorganisms which are not recombinant for the yaaU-ORF, and which do not contain any overexpressed yaaU-ORF, are used as the starting point for the comparison.

These recombinant microorganisms include, in particular, microorganisms of the Enterobacteriaceae family in which a polynucleotide which encodes a polypeptide whose amino acid sequence is at least 90%, in particular at least 95%, preferably at least 98%, are at least 99%, particularly preferably 99.7% and very particularly preferably 100%, identical to an amino acid sequence selected from the group. SEQ ID No. 4, SEQ ID No. 6 and SEQ ID No. 8 is overexpressed.

Preference is given to amino acid sequences which are identical to those from SEQ ID No.4, SEQ ID No. 6 or SEQ ID No. 8.

Said microorganisms contain overexpressed polynucleotides selected from the group:
a) polynucleotide having the nucleotide sequence SEQ ID No. 3, SEQ ID No. 5 or SEQ ID No. 7;
b) polynucleotide having a nucleotide sequence which corresponds to SEQ ID No. 3, SEQ ID No. 5 or SEQ ID No.. 7 within the limits of the degeneracy of the genetic code;
c) polynucleotide sequence having a sequence which hybridizes, under stringent conditions, with the sequence which is complementary to the sequence SEQ ID No. 3, SEQ ID No. 5 or SEQ ID No. 7;
d) polynucleotide having a sequence SEQ ID No. 3, SEQ ID No. 5 or SEQ ID No. 7 which contains functionally neutral sense mutants,
with the polynucleotides encoding a putative sugar transporter.

The invention also relates to a process for fermentatively preparing L-lysine, L-valine or L-threonine, using recombinant microorganisms of the Enterobacteriaceae family which, in particular, already produce said L-amino acids and in which at least the open reading frame (ORF) having the designation yaaU, or nucleotide sequences encoding its gene product, is or are overexpressed.

Preference is given to using the microorganisms which are described.

When L-amino acids or amino acids are mentioned in that which follows, this thereby means one or more amino acids, including their salts, selected from the group L-asparagine, L-threonine; L-serine, L-glutamate, L-glycine, L-alanine, L-cysteine, L-valine, L-methionine, L-proline, L-isoleucine, L-leucine, L-tyrosine, L-phenylalanine, L-histidine, L-lysine, L-tryptophan, L-arginine and L-homoserine. L-threonine is particularly preferred.

In this connection, the term "enhance" describes the increase, in a microorganism, of the intracellular activity or concentration of one or more enzymes or proteins which are encoded by the corresponding DNA, with, for example, the copy number of the gene or genes, or of the ORF or ORFs, being increased by at least one (1) copy; use being made of a strong promoter operatively linked to the gene or of a gene or allele or ORF which encodes a corresponding enzyme or protein having a high activity, and, where appropriate, these measures being combined.

A segment of a nucleotide sequence which encodes, or can encode, a protein and/or a polypeptide or ribonucleic acid to which the prior art is unable to assign any function is designated an open reading frame (ORF). After a function has been assigned to the nucleotide sequence segment in question, this segment is generally referred to as being a gene. Alleles are generally understood as being alternatives forms of a given gene. The forms are distinguished by differences in the nucleotide sequence.

In general, the protein, or the ribonucleic acid, encoded by a nucleotide sequence, i.e. an ORF, a gene or an allele, is designated a gene product.

The enhance measures, in particular overexpression, generally increase the activity or concentration of the corresponding protein by at least 10%, 25%, 50%, 75%, 100%, 150%, 200%, 300%, 400% or 500%, maximally up to 1000% or 2000%, based on that of the wild-type protein or on the activity or concentration of the protein in the parent strain or microorganism which is not recombinant for the corresponding enzyme or protein. The non-recombinant microorganism or parent strain is understood as being the microorganism on which the measures according to the invention are performed.

The invention relates to a process for L-lysine, L-valine or L-threonine by fermenting recombinant microorganisms of the Enterobacteriaceae family, characterized in that
a) said L-amino acid-producing claimed microorganisms, are cultured in a medium under conditions under which the desired L-amino acid is enriched in the medium or in the cells, and
b) the said L-amino acid is isolated, with, optionally, the fermentation broth constituents and/or the biomass remaining in its/their entirety or in portions (from ≥ 0 to 100%) in the isolated product or being removed completely.

The microorganisms which have an overexpressed open reading frame (ORF) designated yaaU, and which are in particular recombinant, are likewise part of the subject matter of the present invention, can produce said L-amino acids from glucose, sucrose, lactose, fructose, maltose, molasses, where appropriate starch and where appropriate cellulose or from glycerol and ethanol. The microorganisms are representatives of the Enterobacteriaceae family and are selected from the genera Escherichia, Erwinia, Providencia and Serratia. The genera Escherichia and Serratia are preferred. The species Escherichia coli may be mentioned, in particular, in the case of the genus Escherichia while the species Serratia marcescens may be mentioned, in particular, in connection with the genus Serratia.

In general, recombinant microorganisms are generated by means of transformation, transduction or conjugation, or a combination of these methods, with a vector which contains the desired ORF, the desired gene, an allele of this ORF or gene, or parts thereof, and/or a promoter which enhances the expression of the ORF or gene. This promoter can be the promoter which has been produced by enhancing mutation from the endogenous regulatory sequence located upstream of the gene or ORF; alternatively, an efficient promotor has been fused to the gene or ORF.

Examples of suitable strains, which, in particular, produce L-threonine, of the genus Escherichia, in particular of the species Escherichia coli are
- Escherichia coli H4581 (EP 0 301 572)
- Escherichia coli KY10935 (Bioscience Biotechnology and Biochemistry 61(11):1877-1882 (1997)
- Escherichia coli VNIIgenetica MG442 (US-A-4278,765)
- Escherichia coli VNIIgenetica M1 (US-A-4,321,325)
- Escherichia coli VNIIgenetica 472T23 (US-A-5,631,157)
- Escherichia coli BKIIM B-3996 (US-A-5,175,107)
- Escherichia coli cat 13 (WO 98/04715)
- Escherichia coli KCCM-10132 (WO 00/09660)

Examples of suitable L-threonine producing strains of the genus Serratia, in particular of the species Serratia marcescens, are
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonine-producing strains of the Enterobacteriaceae family preferably possess, inter alia, one or more of the genetic or phenotypic features selected from the group: resistance to α-amino-p-hydroxyvaleric acid, resistance to thialysine, resistance to ethionine, resistance to α-methylserine, resistance to diaminosuccinic acid, resistance to α-aminobutyric acid, resistance to borrelidin, resistance to cyclopentanecarboxylic acid, resistance to rifampicin, resistance to valine analogs such as valine hydroxamate, resistance to purine analogs, such as 6-dimethylaminopurine, requirement for L-methionine, possible partial and compensatable requirement for L-isoleucine, requirement for mesodiaminopimelic acid, auxotrophy in regard to threonine-containing dipeptides, resistance to L-threonine, resistance to threonine raffinate, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, possible ability to utilize sucrose, enhance of the threonine operon, enhance of homoserine dehydrogenase I-aspartate kinase I, preferably of the feedback-resistant form, enhance of homoserine kinase, enhance of threonine synthase, enhance of aspartate kinase, possibly of the feedback-resistant form, enhance of aspartate semialdehyde dehydrogenase, enhance of phosphoenolpyruvate carboxylase, possibly of the feedback-resistant form, enhance of phosphoenolpyruvate synthase, enhance of transhydrogenase, enhance of the RhtB gene product, enhance of the RhtC gene product, enhance of the YfiK gene product, enhance of a pyruvate carboxylase and attentuation of acetic acid formation.

It has been found that, following overexpression of the gene or the open reading frame (ORF) yaaU, or its alleles, microorganisms of the Enterobacteriaceae family produce L-lysine, L-valine or L-threonine, in an improved manner.

The nucleotide sequences of the Escherichia coli genes or open reading frames (ORFs) belong to the prior art and can be obtained from the Escherichia coli genome sequence published by Blattner et al. (Science 277: 1453-1462 (1997)). It is known that endogenous enzymes (methionine aminipeptidase) are able to cleave off the N-terminal amino acid methionine.

The nucleotide sequences for the yaaU-ORF from Shigella flexneri and Salmonella typhimirium, which likewise belong to the Enterobacteriaceae family, have also been disclosed.

The yaaU ORF of Escherichia coli K12 is described, inter alia, by the following data:

| | |
|---|---|
| Designation: | open reading frame |
| Function: | annotated as a putative transport protein, a membrane transporter of the MFS (major facilitator superfamily) family. The transported substrates vary greatly; the MFS family contains monosaccharide, disaccharide and oligosaccharide transporters, potassium and amino acid transporters, transporters for intermediates of the tricarboxylic acid cycle and also transporters which pump antibiotics out of the cells. |
| Description: | the open reading frame yaaU encodes a 48.7 KDa protein; the isolelectric point is 8.8; when located in the chromosome, yaaU is present, for example in the case of Escherichia coli K12 MG1655, in the intergenic region of the genes carB, encoding the long chain of carbamoyl phosphate synthase, and kefC, encoding a (K(+)/H(+)glutathione-regulated potassium efflux system protein; |
| Reference: | Blattner et al.; Science 277(5331): 1453-1474 (1997) |
| Accession No.: | AE000114 |
| Alternative gene name: | B0045 |

The nucleic acid sequences can be obtained from the databases belonging to the National Center for Biotechnology Information (NCBI) of the National Library of Medicine (Bethesda, MD, USA), the nucleic acid sequence database of the European Molecular Biology Laboratories (EMBL, Heidelberg, Germany or Cambridge, UK) or the Japanese DNA database (DDBJ, Mishima, Japan).

For the sake of greater clarity, the known nucleotide sequence of the Escherichia coli yaaU-ORF is given in SEQ ID No. 3 and the known sequences for the yaaU-ORF of Shigella flexneri (AE015041) and Salmonella typhimurium (AE008697) are given under SEQ ID No. 5 and, respectively, SEQ ID No. 7. The amino acid sequences of the proteins encoded by these reading frames are depicted as SEQ ID No. 4, SEQ ID No. 6 and, respectively, SEQ ID No. 8.

The open reading frames described in the passages indicated can be used in accordance with the invention. In addition, it is possible to use alleles of the genes or open reading frames, which result from the degeneracy of the genetic code or as a consequence of functionally neutral sense mutations. Preference is given to using endogenous genes or endogenous open reading frames.

"Endogenous genes" or "endogenous nucleotide sequences" are understood as being the genes or open reading frames or alleles or nucleotide sequences which are present in a species population.

The suitable alleles of the yaaU-ORF, which contain functionally neutral sense mutations, include, inter alia, those which lead to at most 50 or to at most 40 or to at most 30 or to at most 20, preferably to at most 10 or to at most 5, very particularly preferably to at most 3 or to at most 2, or to at least one, conservative amino acid substitution in the protein which they encode.

In the case of the aromatic amino acids, the substitutions are said to be conservative when phenylalanine, tryptophan and tyrosine are substituted for each other. In the case of the hydrophobic amino acids, the substitutions are said to be conservative when leucine, isoleucine and valine are substituted for each other. In the case of the polar amino acids, the substitutions are said to be conservative when glutamine and asparagine are substituted for each other. In the case of the basic amino acids, the substitutions are said to be conservative when arginine, lysine and histidine are substituted for each other. In the case of the acid amino acids, the substitutions are said to be conservative when aspartic acid and glutamic acid are substituted for each other. In the case of the hydroxyl group-containing amino acids, the substitutions are said to be conservative when serine and threonine are substituted for each other.

In the same way, it is also possible to use nucleotide sequences which encode variants of said proteins, which variants additionally contain an extension or truncation by at least one (1) amino acid at the N terminus or C terminus. This extension or truncation amounts to not more than 50, 40, 30, 20, 10, 5, 3 or 2 amino acids or amino acid residues.

The suitable alleles also include those which encode proteins in which at least one (1) amino acid has been inserted or deleted. The maximum number of such changes, termed indels, can affect 2, 3, 5, 10, 20, but in no case more than 30, amino acids.

The suitable alleles furthermore include those which can be obtained by means of hybridization, in particular under stringent conditions, using SEQ ID No. 3, SEQ ID No. 5 or SEQ ID No. 7 or parts thereof, in particular the coding regions or the sequences which are complementary thereto.

The skilled person finds instructions for identifying DNA sequences by means of hybridization in, inter alia, the manual "The DIG System Users Guide for Filter Hybridization" supplied by Boehringer Mannheim GmbH (Mannheim, Germany, 1993) and Liebl et al. (International Journal of Systematic Bacteriology 41: 255-260 (2991)). The hybridization takes place under stringent conditions, that is the only hybrids formed are those in which the probe and target sequence, i.e. the polynucleotides treated with the probe, are at least 80% identical. It is known that the stringency of the hybridization, including the washing steps, is influenced and/or determined by varying the buffer composition, the temperature and the salt concentration. In general, the hybridization reaction is carried out at a stringency which is relatively low as compared with that of the washing steps (Hybaid Hybridization Guide, Hybaid Limited, Teddington, UK, 1996).

For example, a buffer corresponding to 5x SSC buffer can be used for the hybridization reaction at a temperature of approx. 50°C - 68°C. Under these conditions, probes can also hybridize with polynucleotides which possess less than 70% identity with the sequence of the probe. These hybrids are less stable and are removed by washing under stringent conditions. This can be achieved, for example, by lowering the salt concentration down to 2x SSC and, where appropriate, subsequently to 0.5x SSC (The DIG System User's Guide for Filter Hybridization, Boehringer Mannheim, Mannheim, Germany, 1995) with the temperature being adjusted to approx. 50°C - 68°C, approx. 52°C - 68°C, approx. 54°C - 68°C, approx. 56°C - 68°C, approx. 58°C - 68°C, approx. 60°C - 68°C, approx. 62°C - 68°C, approx. 64°C - 68°C, approx. 66°C - 68°C. Temperature ranges of approx. 64°C - 68°C or approx. 66°C - 68°C are preferred. It is possible, where appropriate, to lower the salt concentration down to a concentration corresponding to 0.2x SSC or 0.1x SSC. By means of increasing the hybridization temperature stepwise, in steps of approx. 1 - 2°C, from 50°C to 68°C, it is possible to isolate polynucleotide fragments which, for example, possess at least 80%, or at least 90%, at least 91%, at least 92%, at least 93%, at least 94%, at least 95%, at least 96%, at least 97%, at least 98%, or at least 99%, identity with the sequence of the probe employed or with the nucleotide sequences shown in SEQ ID No. 3, SEQ ID No. 5 or SEQ ID No. 7. Additional instructions for the hybridization can be obtained commercially in the form of what are termed kits (e.g. DIG Easy Hyb from Roche Diagnostics GmbH, Mannheim, Germany, Catalog No. 1603558). The nucleotide sequences which are thus obtained encode polypeptides which possess at least 90%, in particular at least 95%, preferably at least 98% or at least 99%, very particularly preferably 99.7%, identity with the amino acid sequences depicted in SEQ ID No. 4, SEQ ID No. 6 or SEQ ID No. 8.

In order to achieve enhance, it is possible, for example, to increase the expression of the genes or open reading frames or alleles or to increase the catalytic properties of the protein. Both measures can be combined, where appropriate.

In order to achieve overexpression, the copy number of the corresponding genes or open reading frames can be increased, for example, or the promoter region and regulatory region or the ribosome binding site which is located upstream of the structural gene can be mutated. Expression cassettes which are incorporated upstream of the structural gene act in the same manner. It is also possible to increase expression during the course of the fermentative L-threonine production by incorporating inducible promoters; in addition, using promoters for gene expression which permits a different chronological gene expression can also be advantageous. Expression is likewise improved by means of measures for extending the lifespan of the mRNA. Furthermore, the enzyme activity is also enhanced by preventing the enzyme protein from being broken down. The ORFs, genes or gene constructs can either be present in plasmids having different copy numbers or be integrated, and amplified, in the chromosome. Alternatively, overexpression of the genes concerned can also be achieved by altering the composition of the media and the conduct of the culture.

Methods for overexpression are adequately described in the prior art, for example in Makrides et al. (Microbiological Reviews 60 (3), 512-538 (1996)). Using vectors increases the copy number by at least one (1) copy. The vectors used can be plasmids as described, for example, in US 5,538,873. The vectors used can also be phages, for example phage Mu, as described in EP 0332448, or phage lambda (λ). The copy number can also be increased by incorporating an additional copy into another site in the chromosome, for example in to the att site of phage λ (Yu and Court, Gene 223, 77-81 (1998)). US 5,939,307 reports that it was possible to increase the expression by incorporating expression cassettes or promoters, such as the tac promoter, the trp promoter, the lpp promoter, or the P_{L} promoter or P_{R} promoter of phage λ, upstream, for example, of the chromosomal threonine operon. In the same way, it is possible to use the phage T7 promoters, the gearbox promoters or the nar promoter. Such expression cassettes or promoters can also be used, as described in EP 0 593 792, to overexpress plasmid-bound genes. Using the lacI^{Q} allele in turn makes it possible to control the expression of plasmid-bound genes (Glascock and Weickert, Gene 223, 221-231 (1998)). It is furthermore possible for the activity of the promoters to be increased by modifying their sequence by means of one or more nucleotide substitutions, by means of (an) insertion(s) and/or by means of (a) deletion(s). A different chronological gene expression can be achieved, for example, as described in Walker et al. (Journal of Bacteriology 181: 1269-80 (1999)), by using the growth phase-dependent fis promoter.

The skilled person can find general instructions in this regard in, inter alia, Chang and Cohen (Journal of Bacteriology 134: 1141-1156 (1978)), Hartley and Gregori (Gene 13: 347-353 (1981)), Amann and Brosius (Gene 40: 183-190 (1985)), de Broer et al. (Proceedings of the National Academy of Sciences of the United States of America 80: 21-25 (1983)), LaVallie et al. (BIO/TECHNOLOGY 11: 187-193 (1993)), in PCT/US97/13359, Llosa et al. (Plasmid 26: 222-224 (1991)), Quandt and Klipp (Gene 80: 161-169 (1989)), Hamilton et al. (Journal of Bacteriology 171: 4617-4622 (1989)), Jensen and Hammer (Biotechnology and Bioengineering 58: 191-195 (1998)) and known textbooks of genetics and molecular biology.

Plasmid vectors which can be replicated in Enterobacteriaceae, such as pACYC184-derived cloning vectors (Bartolomé et al.; Gene 102: 75-78 (1991)), pTrc99A (Amann et al.; Gene 69: 301-315 (1988)) or pSC101 derivatives (Vocke and Bastia; Proceedings of the National Academy of Sciences USA 80(21): 6557-6561- (1983)) can be used. In a process according to the invention, it is possible to use a strain which is transformed with a plasmid vector which carries at least one nucleotide sequence, or allele, which encodes the yaaU ORF or its gene product.

The term "transformation" is understood as meaning the uptake of an isolated nucleic acid by a host (microorganism).

It is also possible to use sequence exchange (Hamilton et al.; Journal of Bacteriology 171: 4617-4622 (1989)), conjugation or transduction to transfer mutations, which affect the expression of the given genes or open reading frames, into different strains.

More detailed explanations of the concepts of genetics and molecular biology can be found in known textbooks of genetics and molecular biology such as the textbook by Birge (Bacterial and Bacteriophage Genetics, 4th ed., Springer Verlag, New York (USA), 2000) or the textbook by Berg, Tymoczko and Stryer (Biochemistry, 5th ed., Freeman and Company, New York (USA), 2002) or the manual by Sambrook et al. (Molecular Cloning, A Laboratory Manual, (3-Volume Set), Cold Spring Harbor Laboratory Press, Cold Spring Harbor (USA), 2001).

Furthermore, when using strains of the Enterobacteriaceae family to produce L-lysine, L-valine or L-threonine, it can be advantageous, in addition to overexpressing the open reading frame yaaU, to overexpress one or more enzymes of the known threonine biosynthesis pathway or enzymes of anaplerotic metabolism or enzymes for producing reduced nicotinamide adenine dinucleotide phosphate or enzymes of glycolysis or PTS enzymes or enzymes of sulfur metabolism. Using endogenous genes is generally preferred.

Thus, it is possible, for example, to simultaneously-overexpress, one or more of the genes selected from the group
- at least one gene of the thrABC operon encoding aspartate kinase, homoserine dehydrogenase, homoserine kinase and threonine synthase (US-A-4,278,765),
- the pyruvate carboxylase-encoding Corynebacterium glutamicum pyc gene (WO 99/18228),
- the phosphoenolpyruvate synthase-encoding pps gene (Molecular and General Genetics 231(2): 332-336 (1992)),
- the phosphoenolpyruvate carboxylase-encoding ppc gene (WO 02/064808),
- the pntA and pntB genes encoding the subunits of pyridine transhydrogenase (European Journal of Biochemistry 158: 647-653 (1986)),
- the rhtB gene encoding the homoserine resistance-mediating protein (EP-A-0 994 190),
- the rhtC gene encoding the threonine resistance-mediating protein (EP-A-1 013 765),
- the threonine export carrier protein-encoding Corynebacterium glutamicum thrE gene (WO 01/92545),
- the glutamate dehydrogenase-encoding gdhA gene (Nucleic Acids Research 11: 5257-5266 (1983); Gene 23: 199-209 (1983)),
- the phosphoglucomutase-encoding pgm gene (WO 03/004598),
- the fructose biphosphate aldolase-encoding fba gene (WO 03/004664),
- the ptsHIcrr operon ptsH gene encoding the phosphohistidine protein hexose phosphotransferase of the PTS phosphotransferase system (WO 03/004674),
- the ptsHIcrr operon ptsI gene encoding enzyme I of the PTS phosphotransferase system (WO 03/004674),
- the ptsHIcrr operon crr gene encoding the glucose-specific IIA component of the PTS phosphotransferase system (WO 03/004674),
- the ptsG gene encoding the glucose-specific IIBC component (WO 03/004670),
- the lrp gene encoding the regulator of the leucine regulon (WO 03/004665),
- the fadR gene encoding the regulator of the fad regulon (WO 03/038106),
- the iclR gene encoding the regulator of central intermediary metabolism (WO 03/038106),
- the ahpCF operon ahpC gene encoding the small subunit of alkyl hydroperoxide reductase (WO 03/004663),
- the ahpCF operon ahpF gene encoding the large subunit of alkyl hydroperoxide reductase (WO 03/004663),
- the cysteine synthase A-encoding cysK gene (WO 03/006666),
- the cysB gene encoding the regulator of the cys regulon (WO 03/006666),
- the cysJIH operon cysJ gene encoding the NADPH sulfite reductase flavoprotein (WO 03/006666),
- the cysJIH operon cysI gene encoding the NADPH sulfite reductase hemoprotein (WO 03/006666),
- the adenylyl sulfate reductase-encoding cysJIH operon cysH gene (WO 03/006666),
- the rseABC operon rseA gene encoding a membrane protein which possesses anti-sigmaE activity (WO 03/008612),
- the rseABC operon rseC gene encoding a global regulator of the sigmaE factor (WO 03/008612),
- the sucABCD operon sucA gene encoding the decarboxylase subunit of 2-ketoglutarate dehydrogenase (WO 03/008614),
- the sucABCD operon sucB gene encoding the dihydrolipoyl-transsuccinase E2 subunit of 2-ketoglutarate dehydrogenase (WO 03/008614),
- the suc ABCD operon sucC gene encoding the β-subunit of succinyl-CoA synthetase (WO 03/008615),
- the sucABCD operon sucD gene encoding the α-subunit of succinyl-CoA synthetase (WO 03/008615),
- the aceE gene encoding the E1 component of the pyruvate dehydrogenase complex (WO 03/076635),
- the aceF gene encoding the E2 component of the pyruvate dehydrogenase complex (WO 03/076635),
- the rseB gene encoding the regulator of the SigmaE *factor activity (*Molecular Microbiology 24(2): 355-371 (1997))
- the gene product of the Escherichia coli yodA open reading frame (ORF) (Accession Number AE000288 of the National Center for Biotechnology Information (NCBI, Bethesda, MD, USA, Die10361192.4)).

Furthermore, for the purpose of producing L-amino acids, in particular L-threonine, it can be advantageous, in addition to enhancing the open reading frame yaaU, to attenuate, in particular eliminate or reduce the expression of one or more of the genes selected from the group
- the threonine dehydrogenase-encoding tdh gene (Journal of Bacteriology 169: 4716-4721 (1987)),
- the malate dehydrogenase (E.C. 1.1.1.37)-encoding mdh gene (Archives in Microbiology 149: 36-42 (19987)),
- the gene product of the Escherichia coli yjfA open reading frame (ORF) (Accession Number AAC77180 of the National Center for Biotechnology Information (NCBI, Bethesda, MD, USA, (WO 02/29080)),
- the gene product of the Escherichia coli ytfP open reading frame (ORF) (Accession Number AAC77179 of the National Center for Biotechnology Information (NCBI, Bethesda, MD, USA, WO 02/29080)),
- the pckA gene encoding the enzyme phosphoenolpyruvate carboxykinase (WO 02/29080),
- the pyruvate oxidase-encoding poxB gene (WO 02/36797),
- the dgsA gene (WO 02/081721), which is also known under the name mlc gene, encoding the DgsA regulator of the phosphotransferase system,
- the fruR gene (WO 02/081698), which is also known under the name cra gene, encoding the fructose repressor,
- the rpoS gene (WO 01/05939), which is also known under the name katF gene, encoding the sigma³⁸ factor, and
- the aspartate ammonium lyase-encoding aspA gene (WO 031008603).

In this context, the term "attenuation" describes the reduction or abolition, in a microorganism, of the intracellular activity or concentration of one or more enzymes or proteins which are encoded by the corresponding DNA, by, for example, using a weaker promoter than in the parent strain or microorganism not recombinant for the corresponding enzyme or protein, or a gene or allele which encodes a corresponding enzyme or protein having a lower activity, or inactivating the corresponding enzyme or protein, or the open reading frame or gene, and, where appropriate, combining these measures.

In general, the attenuation measures lower the activity or concentration of the corresponding protein down to from 0 to 75%, from 0 to 50%, from 0 to 25%, from 0 to 10% or from 0 to 5% of the activity or concentration of the wild-type protein or of the activity or concentration of the protein for the parent strain or microorganism which is not recombinant for the corresponding enzyme or protein. The parent strain or microorganism which is not recombinant is understood as being the microorganism on which the measures according to the invention are performed.

In order to achieve an attenuation, for example the expression of the genes or open reading frames, or the catalytic properties of the enzyme proteins, can be reduced or abolished. Where appropriate, both measures can be combined.

The gene expression can be reduced by carrying out the culture in a suitable manner, by genetically altering (mutating) the signal structures for the gene expression or by means of the antisense RNA technique. Signal structures for the gene expression are, for example, repressor genes, activator genes, operators, promoters, attenuators, ribosome binding sites, the start codon and terminators. The skilled person can find information in this regard in, inter alia and for example, Jensen and Hammer (Biotechnology and Bioengineering 58: 191-195 (1998)), in Carrier and Keasling (Biotechnology Progress 15: 58-64 (1999)), in Franch and Gerdes (Current Opinion in Microbiology 3: 159-164 (2000)) and in well known textbooks of genetics and molecular biology such as the textbook by Knippers ("Molekulare Genetik [Molecular Genetics]", 6th edition, Georg Thieme Verlag, Stuttgart, Germany, 1995) or that by Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art. Examples which may be mentioned are the articles by Qiu and Goodman (Journal of Biological Chemistry 272: 8611-8617 (1997)), Yano et al. (Proceedings of the National Academy of Sciences of the United States of America 95: 5511-5515 (1998)) and Wente and Schachmann (Journal of Biological Chemistry 266: 20833-20839 (1991)). Summaries can be found in known textbooks of genetics and molecular biology, such as that by Hagemann ("Allgemeine Genetik [General Genetics]", Gustav Fischer Verlag, Stuttgart, 1986) .

Mutations which come into consideration are transitions, transversions, insertions and deletions of at least one (1) base pair or nucleotide. Depending on the effect of the mutation-elicited amino acid substitution on the enzyme activity, reference is made to missense mutations or to nonsense mutations. A missense mutation leads to the replacement of a given amino acid in a protein with a different amino acid, with the amino acid replacement in particular being non-conservative. This thereby impairs the functional ability or activity of the protein and reduces it down to a value of from 0 to 75%, 0 to 50%, 0 to 25%, 0 to 10% or 0 to 5%. A nonsense mutation leads to a stop codon in the coding region of the gene and thus to premature termination of the translation. Insertions or deletions of at least one base pair in a gene lead to frame shift mutations which in turn result in incorrect amino acids being incorporated or in the translation being prematurely terminated. If a stop codon is formed in the coding region as a consequence of the mutation, this then also leads to translation being terminated prematurely. Deletions of at least one (1) or more codons typically also lead to complete loss of the enzyme activity.

Directions for generating these mutations belong to the prior art and can be obtained from known textbooks of genetics and molecular biology such as the textbook by Knippers ("Molekulare Genetik [Molecular Genetics]", 6th edition, Georg Thieme Verlag, Stuttgart, Germany, 1995), that by Winnacker "Gene und Klone, [Genes and Clones]", VHC Verlagsgesellschaft, Weinheim, Germany, 1990) or that by Hagemann ("Allgemeine Genetik [General Genetics]", Gustav Fischer Verlag, Stuttgart, 1986).

Suitable mutations in the genes can be incorporated into suitable strains by means of gene or allele exchange. A customary method is the method, described by Hamilton et al. (Journal of Bacteriology 171: 4617-4622 (1989)), of gene exchange using a conditionally replicating p*SC101* derivative pMAK705. Other methods described in the prior art, such as that of Martinez-Morales et al. (Journal of Bacteriology 181: 7143-7148 (1999)) or that of Boyd et al. (Journal of Bacteriology 182: 842-847 (2000)), can also be used.

It is likewise possible to transfer mutations in the relevant genes, or mutations which effect the expression of the relevant genes or open reading frames, into different strains by means of conjugation or transduction.

Furthermore, for the purpose of producing L-lysine, L-valine or L-threonine, it can be advantageous, in addition to enhancing the open reading frame yaaU, to eliminate undesirable side-reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms which are prepared in accordance with the invention can be cultured in a batch process, in a fed-batch process, in a repeated fed-batch process or in a continuous process (DE102004028859.3 or US 5,763,230). Known culturing methods are summarized in the textbook by Chmiel (Bioprozesstechnik 1. Einführung in die Bioverfahrenstechnik [Bioprocess technology 1. Introduction to bioprocess technology] (Gustav Fischer Verlag, Stuttgart, 1991)) or in the textbook by Storhas (Bioreaktoren und periphere Einrichtungen [Bioreactors and peripheral installations] (Vieweg Verlag, Brunswick/Wiesbaden, 1994)).

The culture medium to be used must satisfy the demands of the given strains in an appropriate manner. The American Society for Bacteriology manual "Manual of Methods for General Bacteriology" (Washington D.C., USA, 1981) contains descriptions of media for culturing a variety of microorganisms.

Sugars and carbohydrates, such as glucose, sucrose, lactose, fructose, maltose, molasses, starch and, where appropriate, cellulose, oils and fats, such as soybean oil, sunflower oil, peanut oil and coconut fat, fatty acids, such as palmitic acid, stearic acid and linoleic acid, alcohols, such as glycerol and ethanol, and organic acids, such as acetic acid, may be used as the carbon source. These substances may be used individually or as a mixture.

Organic nitrogen-containing compounds, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate, may be used as the nitrogen source. The nitrogen sources may be used individually or as a mixture.

Phosphoric acid, potassium dihydrogen phosphate or dipotassium hydrogen phosphate, or the corresponding sodium-containing salts, may be used as the phosphorus source. In addition, the culture medium must contain salts of metals, such as magnesium sulfate or iron sulfate, which are required for growth. Finally, essential growth promoters, such as amino acids and vitamins, may be used in addition to the abovementioned substances. Suitable precursors can also be added to the culture medium. Said ingredients may be added to the culture in the form of a one-off mixture or suitably fed in during the culture.

The fermentation is generally carried out at a pH of from 5.5 to 9.0, in particular of from 6.0 to 8.0. Basic compounds, such as sodium hydroxide, potassium hydroxide, ammonia or ammonia water, or acidic compounds, such as phosphoric acid or sulfuric acid, are used in a suitable manner for controlling the pH of the culture. Antifoamants, such as fatty acid polyglycol esters, can be used for controlling foaming. Suitable selectively acting substances, for example antibiotics, can be added to the medium in order to maintain the stability of plasmids. Oxygen or oxygen-containing gas mixtures, such as air, are passed into the culture in order to maintain aerobic conditions. The temperature of the culture is normally from 25°C to 45°C and preferably from 30°C to 40°C. The culture is continued until a maximum of L-amino acids or L-threonine has been formed. This objective is normally reached within 10 to 160 hours.

L-amino acids can be analyzed by means of anion exchange chromatography followed by derivatization with ninhydrin, as described in Spackman et al. (Analytical Chemistry 30: 1190-1206 (1958)), or by means of reversed phase HPLC, so as described in Lindroth et al. (Analytical Chemistry 51: 1167-1174 (1979)).

The process according to the invention can be used for fermentatively preparing L-amino acids, such as L-threonine, L-valine, and L-lysine, in particular L-threonine.

The following microorganism was deposited in the Deutsche Sammlung für Mikroorganismen und Zellkulturen [German collection of microorganisms and cell cultures] (DSMZ, Brunswick, Germany) in accordance with the Budapest Treaty:
- Escherichia coli strain E. coli MG442 as DSM 16574.

The present invention is explained in more detail below with the aid of implementation examples.

Minimal (M9) and complete (LB) media used for Escherichia coli are described by J.H. Miller (A short course in bacterial genetics (1992), Cold Spring Harbor Laboratory Press). The isolation of plasmid DNA from Escherichia coli, and also all techniques for restricting, ligating and treating with Klenow phosphatase and alkali phosphatase, are carried out as described in Sambrook et al. (Molecular Cloning - A Laboratory Manual (1989) Cold Spring Harbor Laboratory Press). Unless otherwise indicated, Escherichia coli are transformed as described in Chung et al. (Proceedings of the National Academy of Sciences of the United States of America 86: 2172-2175 (1989)).

The incubation temperature when preparing strains and transformants is 37°C.

### Example 1

Constructing the expression plasmid pTrc99AyaaU

The E. coli K12 yaaU ORF is amplified using the polymerase chain reaction (PCR) and synthetic oligonucleotides. PCR primers are synthesized (MWG Biotech, Ebersberg, Deutschland) on the basis of the nucleotide sequence of the yaaU ORF in E. coli K12 MG1655 (Accession Number AE000114), Blattner et al. (Science 277: 1453-1474 (1997)). The sequences of the primers are modified so as to form recognition sites for restriction enzymes. The EcoRI recognition sequence is selected for the yaaU-ex1 primer and the BamHI recognition sequence is selected for the yaaU-ex2 primer, with these sequences being underlined in the nucleotide sequences shown below:

The E. coli K12 MG1655 chromosomal DNA used for the PCR is isolated using "Qiagen Genomic-tips 100/G" (QIAGEN, Hilden, Germany) in accordance with the manufacturer's instructions. A DNA fragment of approx. 1371 bp in size (SEQ ID No. 3) can be amplified under standard PCR conditions (Innis et al. (1990) PCR Protocols. A Guide to Methods and Applications, Academic Press) using Vent DNA polymerase (New England Biolaps GmbH, Frankfurt, Germany) and the specific primers.

The amplified yaaU fragment is ligated to the vector pCR-Blunt II-TOPO (Zero TOPO TA Cloning Kit, Invitrogen, Groningen, Netherlands) in accordance with the manufacturer's instructions and transformed into the E. coli strain TOP10. Plasmid-harboring cells are selected on LB Agar containing 50 µg of kanamycin/ml. After the plasmid DNA has been isolated, the vector is cleaved with the enzymes EcoRV and EcoRI and, after the cleavage has been checked in a 0.8% agarose gel, designated pCRBluntyaaU.

The vector pCRBluntyaaU is then cleaved with the enzymes EcoRI and BamHI and the yaaU fragment is separated in a 0.8% agarose gel; it is then isolated from the gel (QIAquick Gel Extraction Kit, QIAGEN, Hilden, Germany) and ligated to the vector pTrc99A (Pharmacia Biotech, Uppsala, Sweden) which has been digested with the enzymes BamHI and EcoRI. The E. coli strain XLlBlue MRF' (Stratagene, La Jolla, USA) is transformed with the ligation mixture and plasmid-harboring cells are selected on LB agar containing 50 nag of ampicillin/ml.

The fact that cloning has been successful can be demonstrated, after the plasmid DNA has been isolated, by performing a control cleavage using the enzymes EcoRI/BamHI and EcoRV.

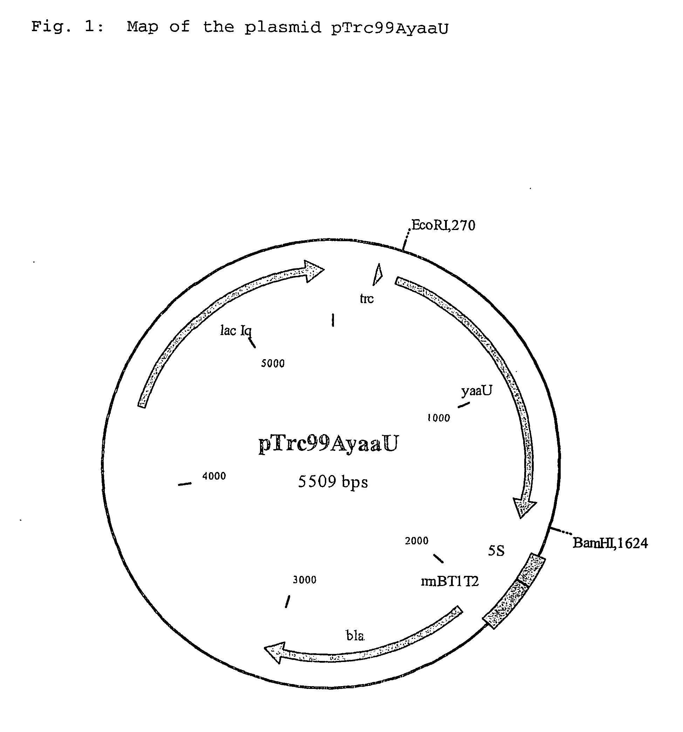

The plasmid is designated pTrc99AyaaU (Figure 1).

### Example 2

### Preparing L-threonine using the strain MG442/pTrc99AyaaU

The L-threonine-producing E. coli strain MG442 is described in the patent specification US-A- 4,278,765 and is deposited in the Russian national collection of industrial microorganisms (VKPM, Moscow, Russia) as CMIM B-1628 and in the Deutsche Sammlung far Mikroorganismen und Zellkulturen [German collection of microorganisms and cell cultures] (DSMZ, Brunswick, Germany), in accordance with the Budapest Treaty, as DSM 16574.)

The strain MG442 is transformed with the expression plasmid pTrc99AyaaU described in example 1, and with the vector pTrc99A, and plasmid-harboring cells are selected on LB agar containing 50 µg of ampicillin/ml. This results in the strains MG442/pTrc99AyaaU and MG442/pTrc99A. Selected individual colonies are then propagated further on minimal medium having the following composition: 3.5 g of Na₂HP0₄*2H₂O/l, 1.5 g of KH₂PO₄/l, 1 g of NH₄Cl/l, 0.1 g of MgSO₄*7H₂O/l, 2 g of glucose/l, 20 g of agar/l, 50 mg of ampicillin/l.

The formation of L-threonine is checked in 10 ml batch cultures which are contained in 100 ml Erlenmeyer flasks. For this, a 10 ml preculture medium of the following composition: 2 g of yeast extract/l, 10 g of (NH₄)ₐSO₄/l, 1 g of KH₂PO₄/l, 0.5 g of MgSO₄*7H₂O/l, 15 g of CaCO₃/l, 20 g of glucose/l, 50 mg of ampicillin/l, is inoculated and incubated, at 37°C and 180 rpm for 16 hours, on a Kühner AG ESR incubator (Birsfelden, Switzerland). In each case 250 µl of this preliminary culture are inoculated over into 10 ml of production medium (25 g of (NH₄)₂SO₄/l, 2 g of KH₂PO₄/l, 1 g of MgSO₄*7H₂O/l, 0.03 g of FeSO₄*7H₂O/l, 0.018 g of MnSO₄*1H₂O/l, 30 g of CaCO₃/l, 20 g of glucose 50 mg of ampicillin/l) and incubated at 37°C for 48 hours. The formation of L-threonine by the starting strain MG442 is checked in the same way with, however, no ampicillin being added to the medium. After the incubation, the optical density (OD) of the culture suspension is determined at a measurement wavelength of 660 nm using a Dr. Lange LP2W photometer (Düsseldorf, Germany).

An Eppendorf-BioTronik amino acid analyzer (Hamburg, Germany) is then used to determine, by means of ion exchange chromatography and post-column reaction involving ninhydrin detection, the concentration of the resulting L-threonine in the culture supernatant, which has been sterilized by filtration.

The result of the experiment is shown in table 1.

**Table 1**

| Strain | OD (660 nm) | L-Threonine g/l |
|---|---|---|
| MG442 | 5.6 | 1.4 |
| MG442/pTrc99A | 3 | 1.3 |
| MG442/pTrc99AyaaU | 4.1 | 2.7 |

### Brief description of the figure:

- Figure 1:: Map of the yaaU gene-containing plasmid pTrc99AyaaU

Length specifications are to be regarded as being approximate. The abbreviations and designations employed have the following meanings:
- bla: gene which encodes resistance to ampicillin
- lac Iq: gene for the trc promoter repressor protein
- trc: trc promoter region, IPTG-inducible
- yaaU: coding region of the yaaU gene
- 5S: 5S rRNA region
- rrnBT: rRNA terminator region

The abbreviations for the restriction enzymes have the following meaning:
- BamHI: restriction endonuclease from Bacillus amyloliquefaciens H
- EcoRI: restriction endonuclease from Escherichia coli RY13
- EcoRV: restriction endonuclease from Escherichia coli B946

## Claims

1. A recombinant microorganism of the Enterobacteriaceae family which contains an overexpressed yaaU ORF, which encodes a polypeptide which is annotated as being a putative sugar transporter, whereby the amino acid sequence of the polypeptide is at least 90% identical to an amino acid sequence selected from the group SEQ ID No. 4, SEQ ID No. 6 and SEQ ID No. 8.

2. A microorganism as claimed in claim 1, **characterized in that** it contains an overexpressed polynucleotide yaaU which is selected from the group:
a) polynucleotide having the nucleotide sequence from SEQ ID No. 3, SEQ ID No. 5 or SEQ ID No. 7;
b) polynucleotide having a nucleotide sequence which corresponds to SEQ ID No. 3, SEQ ID No. 5 or SEQ ID No. 7 within the limits of the degeneracy of the genetic code;
c) polynucleotide sequence having a sequence which hybridizes, under stringent conditions, with the sequence which is complementary to the sequence SEQ ID No. 3, SEQ ID No. 5 or SEQ ID No. 7.

3. A microorganism as claimed in claim 1, **characterized in that** the polypeptide possesses an amino acid sequence which is at least 95% identical to one of the sequences, selected from the group SEQ ID No. 4, SEQ ID No. 6 and SEQ ID No. 8.

4. A microorganism as claimed in claim 1, **characterized in that** the polypeptide possesses the amino acid sequence which is 100% identical to that from SEQ ID No. 4, SEQ ID No. 6 or SEQ ID No.8.

5. A microorganism as claimed in claims 1 to 4, **characterized in that** it is produced by transformation, transduction or conjugation, or a combination of these methods, with a vector which contains said yaaU ORF, an allele of this ORF, or parts thereof, and/or a promotor.

6. A microorganism as claimed in claim 1 or 5, in which the copy number of the yaaU, ORF or the alleles has been increased by at least 1.

7. The microorganism as claimed in claim 6, **characterized in that** the increase in the copy number of said yaaU ORF by at least 1 is achieved by integrating said ORF or the alleles thereof into the chromosome of the microorganism.

8. The microorganism as claimed in claim 6, **characterized in that** the increase in the copy number of said yaaU ORF by at least 1 is achieved by means of a vector which replicates extra-chromosomally.

9. The microorganism as claimed in claim 1 or 2, **characterized in that**
a) the promoter and regulatory region or the ribosomal binding site upstream of said yaaU ORF is mutated, or
b) expression cassettes or promoters are incorporated upstream of the yaaU ORF.

10. The microorganism as claimed in claim 1 or 2, **characterized in that** said polynucleotide yaaU is under the control of a promoter enhancing the expression of the gene.

11. The microorganism as claimed in claims 1 to 10, **characterized in that** enhancing the yaaU ORF increases the concentration or activity of the yaaU gene product (protein) by at least 10%, based on the activity or concentration of the gene product in the parent strain or microorganism not recombinant for the yaaU ORF.

12. The microorganism as claimed in claims 1 to 11, **characterized in that** other genes of the metabolic pathway for the biosynthesis of the desired L-amino acid are also present in overexpressed form.

13. The microorganism as claimed in claim 12, **characterized in that** the microorganism is selected from the genera Escherichia, Erwinia, Providencia and Serratia.

14. The microorganism as claimed in claims 1 to 13, **characterized in that** it produces L-threonine.

15. A process for preparing L-amino acids chosen from the group L-threonine, L-valine, and L-lysine by fermenting recombinant microorganisms of the Enterobacteriaceae family, **characterized in that**
a) said L-amino acid-producing microorganisms according to claims 1-14 are cultured in a medium under conditions under which said L-amino acid is enriched in the medium or in the cells, and
b) the said L-amino acid is isolated, with constituents of the fermentation broth, and/or the biomass remaining in its/their entirety or in portions (from ≥ 0 to 100%) in the isolated product or being removed completely. '

16. The process as claimed in claim 15, **characterized in that**, for the purpose of preparing L-threonine, microorganisms of the Enterobacteriaceae family are fermented in which one or more of the genes selected from the group:
a) at least one gene of the thrABC operon encoding aspartate kinase, homoserine dehydrogenase, homoserine kinase and threonine synthase,
b) the pyruvate carboxylase-encoding Corynebacterium glutamicum pyc gene,
c) the phosphoenolpyruvate synthase-encoding pps gene,
d) the phosphoenolpyruvate carboxylase-encoding ppc gene,
e) the pntA and pntB genes encoding the subunits of pyridine transhydrogenase,
f) the rhtB gene encoding the homoserine resistance-mediating protein,
g) the rhtC gene encoding the threonine resistance-mediating protein,
h) the threonine export carrier protein-encoding Corynebacterium glutamicum thrE gene,
i) the glutamate dehydrogenase-encoding gdhA gene,
j) the phosphoglucomutase-encoding pgm gene,
k) the fructose biphosphate aldolase-encoding fba gene,
l) the ptsH gene encoding the phosphohistidine protein hexose phosphotransferase,
m) the ptsI gene encoding enzyme I of the phosphotransferase system,
n) the crr gene encoding the glucose-specific IIA component,
o) the ptsG gene encoding the glucose-specific IIBC component,
p) the lrp gene encoding the regulator of the leucine regulon,
g) the fadR gene encoding the regulator of the fad regulon,
r) the iclR gene encoding the regulator of central intermediary metabolism,
s) the ahpC gene encoding the small subunit of alkyl hydroperoxide reductase,
t) the ahpF gene encoding the large subunit of alkyl hydroperoxide reductase,
u) the cysteine synthase A-encoding cysK gene,
v) the cysB gene encoding the regulator of the cys regulon,
w) the cysJ gene encoding the NADPH sulfite reductase flavoprotein,
x) the cysI gene encoding the NADPH sulfite reductase hemoprotein,
y) the adenylyl sulfate reductase-encoding cysH gene,
z) the rseA gene encoding a membrane protein which possesses anti-sigmaE activity,
a1) the rseC gene encoding a global regulator of the sigmaE factor
b1) the sucA gene encoding the decarboxylase subunit of 2-ketoglutarate dehydrogenase,
c1) the sucB gene encoding the dihydrolipoyl-transsuccinase E2 subunit of 2-ketoglutarate dehydrogenase,
d1) the sucC gene encoding the β-subunit of succinyl-CoA synthetase,
e1) the sucD gene encoding the α-subunit of succinyl-CoA synthetase,
f1) the aceE gene encoding the E1 component of the pyruvate dehydrogenase complex,
g1) the aceF gene encoding the E2 component of the pyruvate dehydrogenase complex and,
h1) the rseB gene encoding the regulator of the SigmaE factor activity,
i1) the gene product of the Escherichia coli yodA open reading frame (ORF)
is/are additionally, at the same time, overexpressed.

17. The process as claimed in claims 15 to 16, **characterized in that** use is made of microorganisms in which the metabolic pathways which reduce the formation of said L-amino acid are at least partially attenuated.

18. The process as claimed in claim 17, **characterized in that**, for the purpose of preparing L-threonine, microorganisms of the Enterobacteriaceae family are fermented in which one or more of the genes selected from the group:
a) the threonine dehydrogenase-encoding tdh gene,
b) the malate dehydrogenase-encoding mdh gene,
c) the gene product of the Escherichia coli yjfA open reading frame (ORF),
d) the gene product of the Escherichia coli ytfP open reading frame (ORF),
e) the pckA gene encoding the phosphoenolpyruvate carboxykinase,
f) the pyruvate oxidase-encoding poxB gene,
g) the dgsA gene encoding the DgsA regulator of the phosphotransferase system,
h) the fruR gene encoding the fructose repressor,
i) the rpoS gene encoding the sigma³⁸ factor, and,
j) the aspartate ammonium lyase-encoding aspA gene,
is/are additionally, at the same time, attenuated, in particular eliminated, or their expression is reduced.

## Patentansprüche

1. Rekombinanter Mikroorganismus der Familie Enterobacteriaceae, der ein überexprimiertes yaaU-ORF enthält, das ein Polypeptid codiert, zu dem angemerkt wird, dass es sich dabei um einen mutmaßlichen Zuckertransporter handelt, womit die Aminosäuresequenz des Polypeptids mindestens zu 90% identisch mit einer Aminosäuresequenz, ausgewählt aus der Gruppe SEQ ID Nr. 4, SEQ ID Nr. 6 und SEQ ID Nr. 8, ist.

2. Mikroorganismus nach Anspruch 1, **dadurch gekennzeichnet, dass** er ein überexprimiertes Polynukleotid yaaU enthält, das aus der folgenden Gruppe ausgewählt ist:
a) Polynukleotid mit der Nukleotidsequenz aus SEQ ID Nr. 3, SEQ ID Nr. 5 oder SEQ ID Nr. 7;
b) Polynukleotid mit einer Nukleotidsequenz, die im Rahmen der Degeneriertheit des genetischen Codes SEQ ID Nr. 3, SEQ ID Nr. 5 oder SEQ ID Nr. 7 entspricht;
c) Polynukleotidsequenz mit einer Sequenz, die unter stringenten Bedingungen mit der Sequenz, die zu der Sequenz SEQ ID Nr. 3, SEQ ID Nr. 5 oder SEQ ID Nr. 7 komplementär ist, hybridisiert.

3. Mikroorganismus nach Anspruch 1, **dadurch gekennzeichnet, dass** das Polypeptid eine Aminosäuresequenz besitzt, die mindestens zu 95% identisch mit einer der Sequenzen, ausgewählt aus der Gruppe SEQ ID Nr. 4, SEQ ID Nr. 6 und SEQ ID Nr. 8, ist.

4. Mikroorganismus nach Anspruch 1, **dadurch gekennzeichnet, dass** das Polypeptid die Aminosäuresequenz besitzt, die zu 100% identisch mit der aus SEQ ID Nr. 4, SEQ ID Nr. 6 oder SEQ ID Nr. 8 ist.

5. Mikroorganismus nach Anspruch 1 bis 4, **dadurch gekennzeichnet, dass** er durch Transformation, Transduktion oder Konjugation oder durch eine Kombination dieser Verfahren erzeugt wird, mit einem Vektor, der das yaaU-ORF, ein Allel dieses ORF oder Teile davon und/oder einen Promotor enthält.

6. Mikroorganismus nach Anspruch 1 oder 5, bei dem die Kopienzahl des yaaU, ORF oder der Allele um mindestens 1 erhöht wurde.

7. Mikroorganismus nach Anspruch 6, **dadurch gekennzeichnet, dass** die Erhöhung der Kopienzahl des yaaU-ORF um mindestens 1 dadurch erreicht wird, dass das ORF oder die Allele davon in das Chromosom des Mikroorganismus integriert wird bzw. werden.

8. Mikroorganismus nach Anspruch 6, **dadurch gekennzeichnet, dass** die Erhöhung der Kopienzahl des yaaU-ORF um mindestens 1 mittels eines extrachromosomal replizierenden Vektors erreicht wird.

9. Mikroorganismus nach Anspruch 1 oder 2, **dadurch gekennzeichnet, dass**
a) der Promotor- und Regulationsbereich oder die ribosomale Bindungsstelle stromaufwärts von dem yaaU-ORF mutiert ist oder
b) Expressionskassetten oder Promotoren stromaufwärts des yaaU-ORF eingebaut sind.

10. Mikroorganismus nach Anspruch 1 oder 2, **dadurch gekennzeichnet, dass** das Polynukleotid yaaU unter der Kontrolle eines die Expression des Gens verbessernden Promotors steht.

11. Mikroorganismus nach Anspruch 1 bis 10, **dadurch gekennzeichnet, dass** durch die Verbesserung des yaaU-ORF die Konzentration oder Aktivität des yaaU-Genprodukts (-Proteins) um mindestens 10% erhöht wird, bezogen auf die Aktivität bzw. Konzentration des Genprodukts im Ausgangsstamm oder -Mikroorganismus, der für das yaaU-ORF nicht rekombinant ist.

12. Mikroorganismus nach Anspruch 1 bis 11, **dadurch gekennzeichnet, dass** andere Gene des Stoffwechselwegs für die Biosynthese der gewünschten L-Aminosäure ebenso in überexprimierter Form vorliegen.

13. Mikroorganismus nach Anspruch 12, **dadurch gekennzeichnet, dass** der Mikroorganismus aus den Gattungen Escherichia, Erwinia, Providencia und Serratia ausgewählt ist.

14. Mikroorganismus nach Anspruch 1 bis 13, **dadurch gekennzeichnet, dass** er L-Threonin produziert.

15. Verfahren zur Herstellung von L-Aminosäuren, gewählt aus der Gruppe L-Threonin, L-Valin und L-Lysin, durch Fermentieren rekombinanter Mikroorganismen der Familie Enterobacteriaceae, **dadurch gekennzeichnet, dass**
a) die L-Aminosäure produzierenden Mikroorganismen gemäß Anspruch 1-14 in einem Medium unter Bedingungen kultiviert werden, unter denen die L-Aminosäure im Medium oder in den Zellen angereichert wird, und
b) die L-Aminosäure isoliert wird, wobei Bestandteile der Fermentationsbrühe und/oder die Biomasse vollständig oder anteilsweise (von 0 bis 100%) im isolierten Produkt verbleiben oder vollständig abgetrennt werden.

16. Verfahren nach Anspruch 15, **dadurch gekennzeichnet, dass** zwecks Herstellung von L-Threonin Mikroorganismen der Familie Enterobacteriaceae fermentiert werden, bei denen zusätzlich gleichzeitig ein oder mehrere Gene, ausgewählt aus der Gruppe:
a) mindestens ein Gen des Aspartatkinase, Homoserin-Dehydrogenase, Homoserinkinase und Threoninsynthase codierenden thrABC-Operons,
b) das Pyruvatcarboxylase codierende pyc-Gen aus Corynebacterium glutamicum,
c) das Phosphoenolpyruvat-Synthase codierende pps-Gen,
d) das Phosphoenolpyruvat-Carboxylase codierende ppc-Gen,
e) die die Untereinheiten von Pyridin-Transhydrogenase codierenden Gene pntA und pntB,
f) das das Homoserinresistenz vermittelnde Protein codierende rhtB-Gen,
g) das das Threoninresistenz vermittelnde Protein codierende rhtC-Gen,
h) das Threoninexport-Carrier-Protein codierende thrE-Gen aus Corynebacterium glutamicum,
i) das Glutamat-Dehydrogenase codierende gdhA-Gen,
j) das Phosphoglucomutase codierende pgm-Gen,
k) das Fructosebiphosphat-Aldolase codierende fba-Gen,
l) das die Phosphohistidinprotein-Hexose-Phosphotransferase codierende ptsH-Gen,
m) das Enzym I des Phosphotransferase-Systems codierende ptsI-Gen,
n) das die glucosespezifische Komponente IIA codierende crr-Gen,
o) das die glucosespezifische Komponente IIBC codierende ptsG-Gen,
p) das den Regulator des Leucin-Regulons codierende lrp-Gen,
q) das den Regulator des fad-Regulons codierende fadR-Gen,
r) das den Regulator des zentralen Intermediärstoffwechsels codierende iclR-Gen,
s) das die kleine Untereinheit der Alkylhydroperoxid-Reduktase codierende ahpC-Gen,
t) das die große Untereinheit der Alkylhydroperoxid-Reduktase codierende ahpF-Gen,
u) das Cysteinsynthase A codierende cysK-Gen,
v) das den Regulator des cys-Regulons codierende cysB-Gen,
w) das das NADPH-Sulfit-Reduktase-Flavoprotein codierende cysJ-Gen,
x) das das NADPH-Sulfit-Reduktase-Hämoprotein codierende cysI-Gen,
y) das Adenylylsulfat-Reduktase codierende cysH-Gen,
z) das ein Membranprotein mit Anti-sigmaE-Aktivität codierende rseA-Gen,
a1) das eine globalen Regulator des sigmaE-Faktors codierende rseC-Gen,
b1) das die Decarboxylase-Untereinheit von 2-Ketoglutarat-Dehydrogenase codierende sucA-Gen,
c1) das die Dihydrolipoyl-Transsuccinase-E2-Untereinheit von 2-Ketoglutarat-Dehydrogenase codierende sucB-Gen,
d1) das die β-Untereinheit von Succinyl-CoA-Synthetase codierende sucC-Gen,
e1) das die α-Untereinheit von Succinyl-CoA-Synthetase codierende sucD-Gen,
f1) das die Komponente E1 des Pyruvat-Dehydrogenase-Komplexes codierende aceE-Gen,
g1) das die Komponente E2 des Pyruvat-Dehydrogenase-Komplexes codierende aceF-Gen und
h1) das den Regulator der SigmaE-Faktor-Aktivität codierende rseB-Gen,
i1) das Genprodukt des offenen yodA-Leserasters (yodA-ORF) aus Escherichia coli
überexprimiert ist bzw. sind.

17. Verfahren nach Anspruch 15 bis 16, **dadurch gekennzeichnet, dass** Mikroorganismen eingesetzt werden, bei denen die Stoffwechselwege, die die Bildung der L-Aminosäure verringern, wenigstens teilweise abgeschwächt sind.

18. Verfahren nach Anspruch 17, **dadurch gekennzeichnet, dass** zwecks Herstellung von L-Threonin Mikroorganismen der Familie Enterobacteriaceae fermentiert werden, bei denen zusätzlich gleichzeitig ein oder mehrere Gene, ausgewählt aus der Gruppe:
a) das Threonin-Dehydrogenase codierende tdh-Gen,
b) das Malat-Dehydrogenase codierende mdh-Gen,
c) das Genprodukt des offenen yjfA-Leserasters (yjfA-ORF) aus Escherichia coli,
d) das Genprodukt des offenen ytfP-Leserasters (ytfP-ORF) aus Escherichia coli,
e) das die Phosphoenolpyruvat-Carboxykinase codierende pckA-Gen,
f) das Pyruvatoxidase codierende poxB-Gen,
g) das den DgsA-Regulator des Phosphotransferase-Systems codierende dgsA-Gen,
h) das den Fructose-Repressor codierende fruR-Gen,
i) das den sigma³⁸-Faktor codierende rpoS-Gen und
j) das Aspartat-Ammoniumlyase codierende aspA-Gen
abgeschwächt, insbesondere ausgeschaltet ist bzw. sind oder deren Expression vermindert ist.

## Revendications

1. Micro-organisme recombinant de la famille des Enterobacteriacae qui contient un cadre ouvert de lecture (ORF) yaaU surexprimé, qui code pour un polypeptide qui est annoté comme étant un transporteur de glucide putatif, **caractérisé en ce que** la séquence d'acides aminés du polypeptide est à au moins 90 % identique à une séquence d'acides aminés choisie dans le groupe de SEQ ID N° 4, SEQ ID N° 6 et SEQ ID N° 8.

2. Micro-organisme selon la revendication 1, **caractérisé en ce qu'**il contient un polynucléotide surexprimé yaaU qui est choisi dans le groupe de :
a) un polynucléotide ayant la séquence nucléotidique de SEQ ID N° 3, SEQ ID N° 5 ou SEQ ID N° 7 ;
b) un polynucléotide ayant une séquence nucléotidique qui correspond à SEQ ID N° 3, SEQ ID N° 5 ou SEQ ID N° 7 dans les limites de la dégénérescence du code génétique ;
c) une séquence polynucléotidique ayant une séquence qui s'hybride, dans des conditions stringentes, avec la séquence qui est complémentaire de la séquence SEQ ID N° 3, SEQ ID N° 5 ou SEQ ID N° 7.

3. Micro-organisme selon la revendication 1, **caractérisé en ce que** le polypeptide possède une séquence d'acides aminés qui est à au moins 95 % identique à une des séquences, choisies dans le groupe de SEQ ID N° 4, SEQ ID N° 6 et SEQ ID N° 8.

4. Micro-organisme selon la revendication 1, **caractérisé en ce que** le polypeptide possède la séquence d'acides aminés qui est à 100 % identique à celle de SEQ ID N° 4, SEQ ID N° 6 ou SEQ ID N° 8.

5. Micro-organisme selon les revendications 1 à 4, **caractérisé en ce qu'**il est produit par transformation, transduction ou conjugaison, ou une combinaison de ces procédés, avec un vecteur qui contient ledit ORF yaaU, un allèle de cet ORF, ou des parties de celui-ci, et/ou un promoteur.

6. Micro-organisme selon la revendication 1 ou 5, dans lequel le nombre de copies de l'ORF yaaU ou les allèles a été augmenté d'au moins 1.

7. Micro-organisme selon la revendication 6, **caractérisé en ce que** l'augmentation du nombre de copies dudit ORF yaaU d'au moins 1 est obtenue par intégration dudit ORF ou des allèles de celui-ci dans le chromosome du micro-organisme.

8. Micro-organisme selon la revendication 6, **caractérisé en ce que** l'augmentation du nombre de copies dudit ORF yaaU d'au moins 1 est obtenue au moyen d'un vecteur qui se réplique de manière extrachromosomique.

9. Micro-organisme selon la revendication 1 ou 2, **caractérisé en ce que**
a) le promoteur et la région régulatrice ou le site de liaison ribosomique en amont dudit ORF yaaU est muté, ou
b) des cassettes d'expression ou des promoteurs sont incorporés en amont de l'ORF yaaU.

10. Micro-organisme selon la revendication 1 ou 2, **caractérisé en ce que** ledit polynucléotide yaaU est sous le contrôle d'un promoteur augmentant l'expression du gène.

11. Micro-organisme selon les revendications 1 à 10, **caractérisé en ce que** l'augmentation de l'ORF yaaU augmente la concentration ou l'activité du produit génique (protéine) yaaU d'au moins 10 %, sur la base de l'activité ou de la concentration du produit génique dans la souche parente ou le micro-organisme non recombinant pour l'ORF yaaU.

12. Micro-organisme selon les revendications 1 à 11, **caractérisé en ce que** d'autres gènes de la voie métabolique pour la biosynthèse de l'acide aminé L souhaité sont également présents sous forme surexprimée.

13. Micro-organisme selon la revendication 12, **caractérisé en ce que** le micro-organisme est choisi parmi les genres Escherichia, Erwinia, Providencia et Serratia.

14. Micro-organisme selon les revendications 1 à 13, **caractérisé en ce qu'**il produit de la L-thréonine.

15. Procédé pour préparer des acides aminés L choisis dans le groupe de la L-thréonine, la L-valine et la L-lysine par fermentation de micro-organismes recombinants de la famille des Enterobacteriacae, **caractérisé en ce que**
a) lesdits micro-organismes producteurs d'acide aminé L selon les revendications 1 à 14 sont cultivés dans un milieu dans des conditions dans lesquelles ledit acide aminé L est enrichi dans le milieu ou dans les cellules, et
b) ledit acide aminé L est isolé, avec des constituants du bouillon de fermentation, et/ou la biomasse restant dans son intégralité ou partiellement (de ≥ 0 à 100 %) dans le produit isolé ou étant totalement éliminée.

16. Procédé selon la revendication 15, **caractérisé en ce que**, en vue de préparer de la L-thréonine, des micro-organismes de la famille des Enterobacteriacae sont fermentés dans lesquels un ou plusieurs des gènes choisis dans le groupe de :
a) au moins un gène de l'opéron thrABC codant pour l'aspartate kinase, l'homosérine déshydrogénase, l'homosérine kinase et la thréonine synthase,
b) le gène pyc de Corynebacterium glutamicum codant pour la pyruvate carboxylase,
c) le gène pps codant pour la phosphoénolpyruvate synthase,
d) le gène ppc codant pour la phosphoénolpyruvate carboxylase,
e) les gènes pntA et pntB codant pour les sous-unités de la pyridine transhydrogénase,
f) le gène rhtB codant pour la protéine de résistance à l'homosérine,
g) le gène rhtC codant pour la protéine de résistance à la thréonine,
h) le gène thrE de Corynebacterium glutamicum codant pour la protéine transporteuse d'exportation de thréonine,
i) le gène gdhA codant pour la glutamate déshydrogénase,
j) le gène pgm codant pour la phosphoglucomutase,
k) le gène fba codant pour la fructose biphosphate aldolase,
l) le gène ptsH codant pour la phosphohistidine protéine hexose phosphotransférase,
m) le gène ptsI codant pour l'enzyme I du système phosphotransférase,
n) le gène crr codant pour le composant IIA spécifique du glucose,
o) le gène ptsG codant pour le composant IIBC spécifique du glucose,
p) le gène lrp codant pour le régulateur du régulon leucine,
q) le gène fadR codant pour le régulateur du régulon fad,
r) le gène iclR codant pour le régulateur du métabolisme intermédiaire central,
s) le gène ahpC codant pour la petite sous-unité de l'alkyl-hydroperoxyde réductase,
t) le gène ahpF codant pour la grande sous-unité de l'alkyl-hydroperoxyde réductase,
u) le gène cysK codant pour la cystéine synthase A,
v) le gène cysB codant pour le régulateur du régulon cys,
w) le gène cysJ codant pour le flavoprotéine de NADPH sulfite réductase,
x) le gène cysI codant pour l'hémoprotéine de NADPH sulfite réductase,
y) le gène cysH codant pour l'adénylyl-sulfate réductase,
z) le gène rseA codant pour une protéine membranaire qui possède une activité anti-sigmaE,
a1) le gène rseC codant pour un régulateur global du facteur sigmaE,
b1) le gène sucA codant pour la sous-unité décarboxylase de la 2-cétoglutarate déshydrogénase,
c1) le gène sucB codant pour la sous-unité dihydrolipoyl-transsuccinase E2 de la 2-cétoglutarate déshydrogénase,
d1) le gène sucC codant pour la sous-unité β de la succinyl-CoA synthétase,
e1) le gène sucD codant pour la sous-unité α de la succinyl-CoA synthétase,
f1) le gène aceE codant pour le composant E1 du complexe pyruvate déshydrogénase,
g1) le gène aceF codant pour le composant E2 du complexe pyruvate déshydrogénase,
h1) le gène rseB codant pour le régulateur de l'activité du facteur SigmaE,
i1) le produit génique du cadre ouvert de lecture (ORF) yodA d'Escherichia coli
est/sont également, simultanément, surexprimés.

17. Procédé selon les revendications 15 à 16, **caractérisé en ce qu'**il est utilisé des micro-organismes dans lesquels les voies métaboliques qui réduisent la formation dudit acide aminé L sont au moins partiellement atténuées.

18. Procédé selon la revendication 17, **caractérisé en ce que**, en vue de préparer de la L-thréonine, des micro-organismes de la famille des Enterobacteriacae sont fermentés dans lesquels un ou plusieurs des gènes choisis dans le groupe de:
a) le gène tdh codant pour la thréonine déshydrogénase,
b) le gène mdh codant pour la malate déshydrogénase,
c) le produit génique du cadre ouvert de lecture (ORF) yjfA d'Escherichia coli,
d) le produit génique du cadre ouvert de lecture (ORF) ytfP d'Escherichia coli,
e) le gène pckA codant pour la phosphoénolpyruvate carboxylase,
f) le gène poxB codant pour la pyruvate oxydase,
g) le gène dgsA codant pour le régulateur DgsA du système phosphotransférase,
h) le gène fruR codant pour le répresseur de fructose,
i) le gène rpoS codant le facteur sigma³⁸, et,
j) le gène aspA codant pour l'aspartate ammonium lyase,
est/sont également, simultanément, atténués, en particulier éliminés, ou leur expression est réduite.


### SEQUENCE LISTING

<110> Degussa AG
<120> Process for preparing L-amino acids using strains of the Enterobacteriaceae family
<130> 030321 BT
<160> 8
<170> PatentIn version 3.2
<210> 1
   <211> 34
   <212> DNA
   <213> Artificial sequence
<220>
   <221> Primer
   <222> (1)..(34)
   <223> yaaU-ex1
<400> 1
   gatctgaatt ctaaggaata accatgcaac cgtc 34
<210> 2
   <211> 32
   <212> DNA
   <213> Artificial sequence
<220>
   <221> Primer
   <222> (1)..(32)
   <223> yaaU-ex2
<400> 2
   gatctaggat cccaatttac cccattctct gc 32
<210> 3
   <211> 1372
   <212> DNA
   <213> Escherichia coli
<220>
   <221> PCR-product
   <222> (1)..(1372)
   <223> yaaU PCR-product
<220>
   <221> primer_bind
   <222> (1)..(34)
   <223> Primer yaaU-ex1
<220>
   <221> CDS
   <222> (25)..(1356)
   <223> yaaU coding region
<220>
   <221> primer_bind
   <222> (1341)..(1372)
   <223> Primer yaaU-ex2
<400> 3
   gatctgaatt cttaaggaat aacc atg caa ccg tcc aga aac ttt gac gat      51
                              Met Gln Pro Ser Arg Asn Phe Asp Asp
                              1               5
   ctc aaa ttc tcc tct att cac cgc cgc att ttg ctg tgg gga agc ggt      99
   Leu Lys Phe Ser Ser Ile His Arg Arg Ile Leu Leu Trp Gly Ser Gly
   10                  15                  20                  25
   ggt ccg ttt ctg gat ggt tat gta ctg gta atg att ggc gtg gcg ctg     147
   Gly Pro Phe Leu Asp Gly Tyr Val Leu Val Met Ile Gly Val Ala Leu
                   30                  35                  40
   gag caa ctg acg ccg gcg ctg aaa ctg gac gct gac tgg att ggc ttg     195
   Glu Gln Leu Thr Pro Ala Leu Lys Leu Asp Ala Asp Trp Ile Gly Leu
               45                  50                  55
   ctg ggc gcg gga acg ctc gcc ggg ctg ttc gtt ggc aca tcg ctg ttt     243
   Leu Gly Ala Gly Thr Leu Ala Gly Leu Phe Val Gly Thr Ser Leu Phe
           60                  65                  70
   ggt tat att tcc gat aaa gtc gga cgg cgc aaa atg ttc ctc att gat     291
   Gly Tyr Ile Ser Asp Lys Val Gly Arg Arg Lys Met Phe Leu Ile Asp
       75                  80                  85
   atc atc gcc atc ggc gtg ata tcg gtg gcg acg atg ttt gtt tca tcc     339
   Ile Ile Ala Ile Gly Val Ile Ser Val Ala Thr Met Phe Val Ser Ser
   90                  95                  100                 105
   ccc gtc gaa ctg ttg gtg atg cgg gta ctt atc ggc att gtc atc ggt     387
   Pro Val Glu Leu Leu Val Met Arg Val Leu Ile Gly Ile Val Ile Gly
                   110                 115                 120
   gca gat tat ccc atc gcc acc tca atg atc acc gag ttc tcc agt acc     435
   Ala Asp Tyr Pro Ile Ala Thr Ser Met Ile Thr Glu Phe Ser Ser Thr
               125                 130                 135
   cgt cag cgg gcg ttt tcc atc agc ttt att gcc gcg atg tgg tat gtc     483
   Arg Gln Arg Ala Phe Ser Ile Ser Phe Ile Ala Ala Met Trp Tyr Val
           140                 145                 150
   ggc gcg acc tgt gcc gat ctg gtc ggc tac tgg ctt tat gat gtg gaa     531
   Gly Ala Thr Cys Ala Asp Leu Val Gly Tyr Trp Leu Tyr Asp Val Glu
       155                 160                 165
   ggc ggc tgg cgc tgg atg ctg ggt agc gcg gcg atc ccc tgt ttg ttg     579
   Gly Gly Trp Arg Trp Met Leu Gly Ser Ala Ala Ile Pro Cys Leu Leu
   170                 175                 180                 185
   att ttg att ggt cga ttc gaa ctg cct gaa tct ccc cgc tgg tta tta     627
   Ile Leu Ile Gly Arg Phe Glu Leu Pro Glu Ser Pro Arg Trp Leu Leu
                   190                 195                 200
   cgc aaa ggg cga gta aaa gag tgc gaa gag atg atg atc aaa ctg ttt     675
   Arg Lys Gly Arg Val Lys Glu Cys Glu Glu Met Met Ile Lys Leu Phe
               205                 210                 215
   ggc gaa ccg gtg gct ttc gat gaa gag cag ccg cag caa acc cgt ttt     723
   Gly Glu Pro Val Ala Phe Asp Glu Glu Gln Pro Gln Gln Thr Arg Phe
           220                 225                 230
   cgc gat ctg ttt aat cgc cgc cat ttt cct ttt gtt ctg ttt gtt gcc      771
   Arg Asp Leu Phe Asn Arg Arg His Phe Pro Phe Val Leu Phe Val Ala
       235                 240                 245
   gcc atc tgg acc tgc cag gtg atc cca atg ttc gcc att tac acc ttt      819
   Ala Ile Trp Thr Cys Gln Val Ile Pro Met Phe Ala Ile Tyr Thr Phe
   250                 255                 260                 265
   ggc ccg caa atc gtt ggt ttg ttg gga ttg ggg gtt ggc aaa aac gcg      867
   Gly Pro Gln Ile Val Gly Leu Leu Gly Leu Gly Val Gly Lys Asn Ala
                   270                 275                 280
   gca cta ggg aat gtg gtg att agc ctg ttc ttt atg ctc ggc tgt att      915
   Ala Leu Gly Asn Val Val Ile Ser Leu Phe Phe Met Leu Gly Cys Ile
               285                 290                 295
   ccg ccg atg ctg tgg tta aac act gcc gga cgg cgt cca ttg ttg att      963
   Pro Pro Met Leu Trp Leu Asn Thr Ala Gly Arg Arg Pro Leu Leu Ile
           300                 305                 310
   ggc agc ttt gcc atg atg acg ctg gcg ctg gcg gtt ttg ggg cta atc     1011
   Gly Ser Phe Ala Met Met Thr Leu Ala Leu Ala Val Leu Gly Leu Ile
       315                 320                 325
   ccg gat atg ggg atc tgg ctg gta gtg atg gcc ttt gcg gtg tat gcc     1059
   Pro Asp Met Gly Ile Trp Leu Val Val Met Ala Phe Ala Val Tyr Ala
   330                 335                 340                 345
   ttt ttc tct ggc ggg ccg ggt aat ttg cag tgg ctc tat cct aat gaa     1107
   Phe Phe Ser Gly Gly Pro Gly Asn Leu Gln Trp Leu Tyr Pro Asn Glu
                   350                 355                 360
   ctc ttc ccg aca gat atc cgc gcc tct gcc gtg ggc gtg att atg tcc     1155
   Leu Phe Pro Thr Asp Ile Arg Ala Ser Ala Val Gly Val Ile Met Ser
               365                 370                 375
   tta agt cgt att ggc acc att gtt tcg acc tgg gca cta ccg atc ttt     1203
   Leu Ser Arg Ile Gly Thr Ile Val Ser Thr Trp Ala Leu Pro Ile Phe
           380                 385                 390
   atc aat aat tac ggt atc agt aac acg atg cta atg ggg gcg ggt atc     1251
   Ile Asn Asn Tyr Gly Ile Ser Asn Thr Met Leu Met Gly Ala Gly Ile
       395                 400                 405
   tcg ctg ttt ggc ttg ttg att tcc gta gcg ttt gcc ccg gag act cga     1299
   Ser Leu Phe Gly Leu Leu Ile Ser Val Ala Phe Ala Pro Glu Thr Arg
   410                 415                 420                 425
   ggg atg tca ctg gcg cag acc agc aat atg acg atc cgc ggg cag aga     1347
   Gly Met Ser Leu Ala Gln Thr Ser Asn Met Thr Ile Arg Gly Gln Arg
                   430                 435                 440
   atg ggg taa attgggatcc tagatc                                       1372
   Met Gly
<210> 4
   <211> 443
   <212> PRT
   <213> Escherichia coli
<400> 4
   Met Gln Pro Ser Arg Asn Phe Asp Asp Leu Lys Phe Ser Ser Ile His
   1               5                   10                  15
   Arg Arg Ile Leu Leu Trp Gly Ser Gly Gly Pro Phe Leu Asp Gly Tyr
               20                  25                  30
   Val Leu Val Met Ile Gly Val Ala Leu Glu Gln Leu Thr Pro Ala Leu
           35                  40                  45
   Lys Leu Asp Ala Asp Trp Ile Gly Leu Leu Gly Ala Gly Thr Leu Ala
       50                  55                  60
   Gly Leu Phe Val Gly Thr Ser Leu Phe Gly Tyr Ile Ser Asp Lys Val
   65                  70                  75                  80
   Gly Arg Arg Lys Met Phe Leu Ile Asp Ile Ile Ala Ile Gly Val Ile
                   85                  90                  95
   Ser Val Ala Thr Met Phe Val Ser Ser Pro Val Glu Leu Leu Val Met
               100                 105                 110
   Arg Val Leu Ile Gly Ile Val Ile Gly Ala Asp Tyr Pro Ile Ala Thr
           115                 120                 125
   Ser Met Ile Thr Glu Phe Ser Ser Thr Arg Gln Arg Ala Phe Ser Ile
       130                 135                 140
   Ser Phe Ile Ala Ala Met Trp Tyr Val Gly Ala Thr Cys Ala Asp Leu
   145                 150                 155                 160
   Val Gly Tyr Trp Leu Tyr Asp Val Glu Gly Gly Trp Arg Trp Met Leu
                   165                 170                 175
   Gly Ser Ala Ala Ile Pro Cys Leu Leu Ile Leu Ile Gly Arg Phe Glu
               180                 185                 190
   Leu Pro Glu Ser Pro Arg Trp Leu Leu Arg Lys Gly Arg Val Lys Glu
           195                 200                 205
   Cys Glu Glu Met Met Ile Lys Leu Phe Gly Glu Pro Val Ala Phe Asp
       210                 215                 220
   Glu Glu Gln Pro Gln Gln Thr Arg Phe Arg Asp Leu Phe Asn Arg Arg
   225                 230                 235                 240
   His Phe Pro Phe Val Leu Phe Val Ala Ala Ile Trp Thr Cys Gln Val
                   245                 250                 255
   Ile Pro Met Phe Ala Ile Tyr Thr Phe Gly Pro Gln Ile Val Gly Leu
               260                 265                 270
   Leu Gly Leu Gly Val Gly Lys Asn Ala Ala Leu Gly Asn Val Val Ile
   275 280 285
   Ser Leu Phe Phe Met Leu Gly Cys Ile Pro Pro Met Leu Trp Leu Asn
   290 295 300
   Thr Ala Gly Arg Arg Pro Leu Leu Ile Gly Ser Phe Ala Met Met Thr
   305 310 315 320
   Leu Ala Leu Ala Val Leu Gly Leu Ile Pro Asp Met Gly Ile Trp Leu
   325 330 335
   Val Val Met Ala Phe Ala Val Tyr Ala Phe Phe Ser Gly Gly Pro Gly
   340 345 350
   Asn Leu Gln Trp Leu Tyr Pro Asn Glu Leu Phe Pro Thr Asp Ile Arg
   355 360 365
   Ala Ser Ala Val Gly Val Ile Met Ser Leu Ser Arg Ile Gly Thr Ile
   370 375 380
   Val Ser Thr Trp Ala Leu Pro Ile Phe Ile Asn Asn Tyr Gly Ile Ser
   385 390 395 400
   Asn Thr Met Leu Met Gly Ala Gly Ile Ser Leu Phe Gly Leu Leu Ile
   405 410 415
   Ser Val Ala Phe Ala Pro Glu Thr Arg Gly Met Ser Leu Ala Gln Thr
   420 425 430
   Ser Asn Met Thr Ile Arg Gly Gln Arg Met Gly
   435 440
<210> 5
   <211> 1395
   <212> DNA
   <213> Shigella flexneri
<220>
   <221> CDS
   <222> (1)..(1395)
   <223> yaaU coding region
<400> 5
   atg caa ccg tcc aga aac ttt gac gat ctc aaa ttc tcc tct att cac      48
   Met Gln Pro Ser Arg Asn Phe Asp Asp Leu Lys Phe Ser Ser Ile His
   1               5                   10                  15
   cgc cgc att ttg ctg tgg gga agc ggt ggt ccg ttt ctg gat ggt tat      96
   Arg Arg Ile Leu Leu Trp Gly Ser Gly Gly Pro Phe Leu Asp Gly Tyr
               20                  25                  30
   gta ctg gta atg att ggc gtg gcg ctg gag caa ctg act ccg gcg ctg      144
   Val Leu Val Met Ile Gly Val Ala Leu Glu Gln Leu Thr Pro Ala Leu
           35                  40                  45
   aaa ctg gac gct gac tgg att ggc ttg ctg ggc gcg gga acg ctc gcc      192
   Lys Leu Asp Ala Asp Trp Ile Gly Leu Leu Gly Ala Gly Thr Leu Ala
       50                  55                  60
   ggg ctg ttc gtt ggc aca tcg ctg ttt ggc tat atc tcc gat aaa gtc      240
   Gly Leu Phe Val Gly Thr Ser Leu Phe Gly Tyr Ile Ser Asp Lys Val
   65                  70                  75                  80
   gga cgg cgc aaa atg ttc ctc att gat atc atc gcc atc ggc gtg ata      288
   Gly Arg Arg Lys Met Phe Leu Ile Asp Ile Ile Ala Ile Gly Val Ile
                   85                  90                  95
   tcg gtg gcg acg atg tgt gtt tca tcc ccc gtc gaa ctg ttg gtg atg      336
   Ser Val Ala Thr Met Cys Val Ser Ser Pro Val Glu Leu Leu Val Met
               100                 105                 110
   cgg gta ctc atc ggc att gtc atc ggt gca gat tat ccc atc gcc act      384
   Arg Val Leu Ile Gly Ile Val Ile Gly Ala Asp Tyr Pro Ile Ala Thr
           115                 120                 125
   tcg atg atc acc gag ttc tcc agt acc cgt cag cgg gcg ttt tcc atc      432
   Ser Met Ile Thr Glu Phe Ser Ser Thr Arg Gln Arg Ala Phe Ser Ile
       130                 135                 140
   agc ttt att gcc gcg atg tgg tat gtc ggc gcg acc tgc gcc gat ctg      480
   Ser Phe Ile Ala Ala Met Trp Tyr Val Gly Ala Thr Cys Ala Asp Leu
   145                 150                 155                 160
   gtc ggc tac tgg ctt tat gat gtg gaa ggt ggt tgg cgc tgg atg ctg      528
   Val Gly Tyr Trp Leu Tyr Asp Val Glu Gly Gly Trp Arg Trp Met Leu
                   165                 170                 175
   ggt agc gcg gcg atc cct tgt ttg ttg att ttg att ggt cga ttc gaa      576
   Gly Ser Ala Ala Ile Pro Cys Leu Leu Ile Leu Ile Gly Arg Phe Glu
               180                 185                 190
   ctg cca gaa tct ccc cgc tgg tta tta cgc aaa ggg cga gta aaa gag      624
   Leu Pro Glu Ser Pro Arg Trp Leu Leu Arg Lys Gly Arg Val Lys Glu
           195                 200                 205
   tgc gaa gag atg atg ata aaa ctg ttt ggc gaa ccg gtg gct ttc gat      672
   Cys Glu Glu Met Met Ile Lys Leu Phe Gly Glu Pro Val Ala Phe Asp
       210                 215                 220
   gaa gag cag ccg cag caa acc cgt ttt cgc gat ctg ttt aat cgc cgc      720
   Glu Glu Gln Pro Gln Gln Thr Arg Phe Arg Asp Leu Phe Asn Arg Arg
   225                 230                 235                 240
   cat ttt cct ttt gtt ctg ttt gtt gcc gcc atc tgg acc tgc cag gtg      768
   His Phe Pro Phe Val Leu Phe Val Ala Ala Ile Trp Thr Cys Gln Val
                   245                 250                 255
   atc cca atg ttc gcc att tac acc ttt ggc ccg caa atc gtt ggt tcg      816
   Ile Pro Met Phe Ala Ile Tyr Thr Phe Gly Pro Gln Ile Val Gly Ser
               260                 265                 270
   ttg gga ttg ggg gtt ggc aaa aac gcg gca cta ggg aat gtg gtg att      864
   Leu Gly Leu Gly Val Gly Lys Asn Ala Ala Leu Gly Asn Val Val Ile
           275                 280                 285
   agc ctg ttc ttt atg ctc ggc tgt att ccg ccg atg ctg tgg cta aac      912
   Ser Leu Phe Phe Met Leu Gly Cys Ile Pro Pro Met Leu Trp Leu Asn
       290                 295                 300
   act gcc gga cgg cgt cca ttg ttg att ggc agc ttt gcc atg atg acg      960
   Thr Ala Gly Arg Arg Pro Leu Leu Ile Gly Ser Phe Ala Met Met Thr
   305                 310                 315                 320
   ctg gcg ttg gcg gtt ttg ggg cta atc ccg gat atg ggg atc tgg ctg     1008
   Leu Ala Leu Ala Val Leu Gly Leu Ile Pro Asp Met Gly Ile Trp Leu
                   325                 330                 335
   gta gtg atg gcc ttt gcg gtg tat gcc ttt ttc tct ggc ggg ccg ggt     1056
   Val Val Met Ala Phe Ala Val Tyr Ala Phe Phe Ser Gly Gly Pro Gly
               340                 345                 350
   aat ttg cag tgg ctc tat cct aat gaa ctc ttc ccg acg gat atc cgc     1104
   Asn Leu Gln Trp Leu Tyr Pro Asn Glu Leu Phe Pro Thr Asp Ile Arg
           355                 360                 365
   gcc tct gcc gtg ggc gtg att atg tcc tta agt cgt att ggc acc att     1152
   Ala Ser Ala Val Gly Val Ile Met Ser Leu Ser Arg Ile Gly Thr Ile
       370                 375                 380
   gtt tcg acc tgg gca ctg ccg ata ttt atc aat aat tac ggt atc agt     1200
   Val Ser Thr Trp Ala Leu Pro Ile Phe Ile Asn Asn Tyr Gly Ile Ser
   385                 390                 395                 400
   aac acg atg cta atg ggg gcg ggt atc tcg ctg ttt ggc ttg ttg att     1248
   Asn Thr Met Leu Met Gly Ala Gly Ile Ser Leu Phe Gly Leu Leu Ile
                   405                 410                 415
   tcc gta gcg ttt gcc ccg gag act cga gga atg tca ctg gcg cag acc     1296
   Ser Val Ala Phe Ala Pro Glu Thr Arg Gly Met Ser Leu Ala Gln Thr
               420                 425                 430
   agc aat atg acg atc cgc ggg cag aga atg gga tta ttt gtt cag att     1344
   Ser Asn Met Thr Ile Arg Gly Gln Arg Met Gly Leu Phe Val Gln Ile
           435                 440                 445
   tct ctc ttt tct gaa tca ata tta ttg act ata agc cgc gtg aat ata     1392
   Ser Leu Phe Ser Glu Ser Ile Leu Leu Thr Ile Ser Arg Val Asn Ile
       450                 455                 460
   tga                                                                 1395
<210> 6
   <211> 464
   <212> PRT
   <213> Shigella flexneri
<400> 6
   Met Gln Pro Ser Arg Asn Phe Asp Asp Leu Lys Phe Ser Ser Ile His
   1               5                   10                  15
   Arg Arg Ile Leu Leu Trp Gly Ser Gly Gly Pro Phe Leu Asp Gly Tyr
               20                  25                  30
   Val Leu Val Met Ile Gly Val Ala Leu Glu Gln Leu Thr Pro Ala Leu
           35                  40                  45
   Lys Leu Asp Ala Asp Trp Ile Gly Leu Leu Gly Ala Gly Thr Leu Ala
   50 55 60
   Gly Leu Phe Val Gly Thr Ser Leu Phe Gly Tyr Ile Ser Asp Lys Val
   65 70 75 80
   Gly Arg Arg Lys Met Phe Leu Ile Asp Ile Ile Ala Ile Gly Val Ile
   85 90 95
   Ser Val Ala Thr Met Cys Val Ser Ser Pro Val Glu Leu Leu Val Met
   100 105 110
   Arg Val Leu Ile Gly Ile Val Ile Gly Ala Asp Tyr Pro Ile Ala Thr
   115 120 125
   Ser Met Ile Thr Glu Phe Ser Ser Thr Arg Gln Arg Ala Phe Ser Ile
   130 135 140
   Ser Phe Ile Ala Ala Met Trp Tyr Val Gly Ala Thr Cys Ala Asp Leu
   145 150 155 160
   Val Gly Tyr Trp Leu Tyr Asp Val Glu Gly Gly Trp Arg Trp Met Leu
   165 170 175
   Gly Ser Ala Ala Ile Pro Cys Leu Leu Ile Leu Ile Gly Arg Phe Glu
   180 185 190
   Leu Pro Glu Ser Pro Arg Trp Leu Leu Arg Lys Gly Arg Val Lys Glu
   195 200 205
   Cys Glu Glu Met Met Ile Lys Leu Phe Gly Glu Pro Val Ala Phe Asp
   210 215 220
   Glu Glu Gln Pro Gln Gln Thr Arg Phe Arg Asp Leu Phe Asn Arg Arg
   225 230 235 240
   His Phe Pro Phe Val Leu Phe Val Ala Ala Ile Trp Thr Cys Gln Val
   245 250 255
   Ile Pro Met Phe Ala Ile Tyr Thr Phe Gly Pro Gln Ile Val Gly Ser
   260 265 270
   Leu Gly Leu Gly Val Gly Lys Asn Ala Ala Leu Gly Asn Val Val Ile
   275 280 285
   Ser Leu Phe Phe Met Leu Gly Cys Ile Pro Pro Met Leu Trp Leu Asn
   290 295 300
   Thr Ala Gly Arg Arg Pro Leu Leu Ile Gly Ser Phe Ala Met Met Thr
   305                 310                 315                 320
   Leu Ala Leu Ala Val Leu Gly Leu Ile Pro Asp Met Gly Ile Trp Leu
                   325                 330                 335
   Val Val Met Ala Phe Ala Val Tyr Ala Phe Phe Ser Gly Gly Pro Gly
               340                 345                 350
   Asn Leu Gln Trp Leu Tyr Pro Asn Glu Leu Phe Pro Thr Asp Ile Arg
           355                 360                 365
   Ala Ser Ala Val Gly Val Ile Met Ser Leu Ser Arg Ile Gly Thr Ile
       370                 375                 380
   Val Ser Thr Trp Ala Leu Pro Ile Phe Ile Asn Asn Tyr Gly Ile Ser
   385                 390                 395                 400
   Asn Thr Met Leu Met Gly Ala Gly Ile Ser Leu Phe Gly Leu Leu Ile
                   405                 410                 415
   Ser Val Ala Phe Ala Pro Glu Thr Arg Gly Met Ser Leu Ala Gln Thr
               420                 425                 430
   Ser Asn Met Thr Ile Arg Gly Gln Arg Met Gly Leu Phe Val Gln Ile
           435                 440                 445
   Ser Leu Phe Ser Glu Ser Ile Leu Leu Thr Ile Ser Arg Val Asn Ile
       450                 455                 460
<210> 7
   <211> 1341
   <212> DNA
   <213> Salmonella typhimurium
<220>
   <221> CDS
   <222> (1)..(1341)
   <223> yaaU coding region
<400> 7
   atg cag cag ccc agg aac ttt gat gac ctt aaa ttc tcc tcc att cat      48
   Met Gln Gln Pro Arg Asn Phe Asp Asp Leu Lys Phe Ser Ser Ile His
   1               5                   10                  15
   cgc aga att atg ctg tgg gga agc ggc ggc ccg ttc ctt gat ggc tac      96
   Arg Arg Ile Met Leu Trp Gly Ser Gly Gly Pro Phe Leu Asp Gly Tyr
               20                  25                  30
   gtg ctg gtg atc att ggc gtg gcg ctg gaa caa ctg acg ccg cta ttg     144
   Val Leu Val Ile Ile Gly Val Ala Leu Glu Gln Leu Thr Pro Leu Leu
           35                  40                  45
   cac ctt gat gct gaa tgg att ggc gcg ctc ggc gcg gct acg ctt gcc     192
   His Leu Asp Ala Glu Trp Ile Gly Ala Leu Gly Ala Ala Thr Leu Ala
       50                  55                  60
   gga ctg ttt atc ggc acc tcg ttg ttt ggc tat att tgc gat aaa gtc      240
   Gly Leu Phe Ile Gly Thr Ser Leu Phe Gly Tyr Ile Cys Asp Lys Val
   65                  70                  75                  80
   ggg cgg cgt aaa atg ttc ctg ctt gat att atc gct atc ggc gtt atc      288
   Gly Arg Arg Lys Met Phe Leu Leu Asp Ile Ile Ala Ile Gly Val Ile
                   85                  90                  95
   tcc gtg gcg acc atg ttt gtt tcc acg ccg ctt gaa tta ctg gtg atg      336
   Ser Val Ala Thr Met Phe Val Ser Thr Pro Leu Glu Leu Leu Val Met
               100                 105                 110
   cgc gtt tta att ggc att gtt atc ggc gcg gat tac cct atc gct act      384
   Arg Val Leu Ile Gly Ile Val Ile Gly Ala Asp Tyr Pro Ile Ala Thr
           115                 120                 125
   tcc atg att acg gag ttt tcc aac acc cgc cag cgc gcg ttc tcc atc      432
   Ser Met Ile Thr Glu Phe Ser Asn Thr Arg Gln Arg Ala Phe Ser Ile
       130                 135                 140
   ggt ttt atc gcc gcg atg tgg tac gtc ggc gcg acc tgc gcc aac ctg      480
   Gly Phe Ile Ala Ala Met Trp Tyr Val Gly Ala Thr Cys Ala Asn Leu
   145                 150                 155                 160
   gtg ggc tac tgg ctg tat gac atg gaa ggc ggc tgg cgt tgg atg ctt      528
   Val Gly Tyr Trp Leu Tyr Asp Met Glu Gly Gly Trp Arg Trp Met Leu
                   165                 170                 175
   ggc agc gcc ttt atc ccg tgc tta atc att ttg att ggc cgc ttt gac      576
   Gly Ser Ala Phe Ile Pro Cys Leu Ile Ile Leu Ile Gly Arg Phe Asp
               180                 185                 190
   ctg ccg gaa tcg cca cgc tgg cta ttg cga aaa gga cgg gta aaa gag      624
   Leu Pro Glu Ser Pro Arg Trp Leu Leu Arg Lys Gly Arg Val Lys Glu
           195                 200                 205
   tgc gaa caa atg atg atc aaa ctg ttc ggc gaa ccg gtc tgc ttt gac      672
   Cys Glu Gln Met Met Ile Lys Leu Phe Gly Glu Pro Val Cys Phe Asp
       210                 215                 220
   gat gag ctg ccg cag gag acc cgc ttc ctg cag ctg ttt aac cgc cgt      720
   Asp Glu Leu Pro Gln Glu Thr Arg Phe Leu Gln Leu Phe Asn Arg Arg
   225                 230                 235                 240
   cat ttc ccg ttt gta ctg ttc gtt gcg gct atc tgg acc tgt cag gtg      768
   His Phe Pro Phe Val Leu Phe Val Ala Ala Ile Trp Thr Cys Gln Val
                   245                 250                 255
   atc ccg atg ttc gct atc tat acc ttt ggg ccg cag atc gtc ggg tta      816
   Ile Pro Met Phe Ala Ile Tyr Thr Phe Gly Pro Gln Ile Val Gly Leu
               260                 265                 270
   ctc ggc tgg gag cag ggg cgc aac gcg gcg ctt ggc aat gtg gtg att      864
   Leu Gly Trp Glu Gln Gly Arg Asn Ala Ala Leu Gly Asn Val Val Ile
           275                 280                 285
   agc ctc ttt ttt atg ctg ggc tgt ata ccg gcg atg ttc tgg cta aac      912
   Ser Leu Phe Phe Met Leu Gly Cys Ile Pro Ala Met Phe Trp Leu Asn
       290                 295                 300
   agt att ggc cgt cgc cct ctg ctt att ggc agc ttc gcc atg atg act      960
   Ser Ile Gly Arg Arg Pro Leu Leu Ile Gly Ser Phe Ala Met Met Thr
   305                 310                 315                 320
   atc gcg ctg gcg ctg ctg ggg ctg gtg tca aat ctg ggc atc ata ctg      1008
   Ile Ala Leu Ala Leu Leu Gly Leu Val Ser Asn Leu Gly Ile Ile Leu
                   325                 330                 335
   gtc gtg gtg gcg ttt gcg gta tac gcc ttc ttt tcc ggc gga ccg ggc      1056
   Val Val Val Ala Phe Ala Val Tyr Ala Phe Phe Ser Gly Gly Pro Gly
               340                 345                 350
   ata ctg caa tgg ctt tat cct aat gaa ctc ttc ccg acg gat att cgc      1104
   Ile Leu Gln Trp Leu Tyr Pro Asn Glu Leu Phe Pro Thr Asp Ile Arg
           355                 360                 365
   gct tcg gcc gtg ggg gtc atc atg tca tta agc cgc atc gga acc att      1152
   Ala Ser Ala Val Gly Val Ile Met Ser Leu Ser Arg Ile Gly Thr Ile
       370                 375                 380
   gtc tcc acc tgg gca tta ccg att ttt att act cgt tac ggt att aat      1200
   Val Ser Thr Trp Ala Leu Pro Ile Phe Ile Thr Arg Tyr Gly Ile Asn
   385                 390                 395                 400
   aac gtc atg ctg att ggc gcg ttg att tcg tta gtc ggg ctt ggc gta      1248
   Asn Val Met Leu Ile Gly Ala Leu Ile Ser Leu Val Gly Leu Gly Val
                   405                 410                 415
   tcg gtg atg ttt gcc ccg gaa acc cgc ggc ttg acg cta aca cag acc      1296
   Ser Val Met Phe Ala Pro Glu Thr Arg Gly Leu Thr Leu Thr Gln Thr
               420                 425                 430
   gga aac atg act ttg cgc gga acg cca tcg gat aac cct cgt taa          1341
   Gly Asn Met Thr Leu Arg Gly Thr Pro Ser Asp Asn Pro Arg
           435                 440                 445
<210> 8
   <211> 446
   <212> PRT
   <213> Salmonella typhimurium
<400> 8
   Met Gln Gln Pro Arg Asn Phe Asp Asp Leu Lys Phe Ser Ser Ile His
   1               5                   10                  15
   Arg Arg Ile Met Leu Trp Gly Ser Gly Gly Pro Phe Leu Asp Gly Tyr
               20                  25                  30
   Val Leu Val Ile Ile Gly Val Ala Leu Glu Gln Leu Thr Pro Leu Leu
           35                  40                  45
   His Leu Asp Ala Glu Trp Ile Gly Ala Leu Gly Ala Ala Thr Leu Ala
       50                  55                  60
   Gly Leu Phe Ile Gly Thr Ser Leu Phe Gly Tyr Ile Cys Asp Lys Val
   65                  70                  75                  80
   Gly Arg Arg Lys Met Phe Leu Leu Asp Ile Ile Ala Ile Gly Val Ile
                   85                  90                  95
   Ser Val Ala Thr Met Phe Val Ser Thr Pro Leu Glu Leu Leu Val Met
   100 105 110
   Arg Val Leu Ile Gly Ile Val Ile Gly Ala Asp Tyr Pro Ile Ala Thr
   115 120 125
   Ser Met Ile Thr Glu Phe Ser Asn Thr Arg Gln Arg Ala Phe Ser Ile
   130 135 140
   Gly Phe Ile Ala Ala Met Trp Tyr Val Gly Ala Thr Cys Ala Asn Leu
   145 150 155 160
   Val Gly Tyr Trp Leu Tyr Asp Met Glu Gly Gly Trp Arg Trp Met Leu
   165 170 175
   Gly Ser Ala Phe Ile Pro Cys Leu Ile Ile Leu Ile Gly Arg Phe Asp
   180 185 190
   Leu Pro Glu Ser Pro Arg Trp Leu Leu Arg Lys Gly Arg Val Lys Glu
   195 200 205
   Cys Glu Gln Met Met Ile Lys Leu Phe Gly Glu Pro Val Cys Phe Asp
   210 215 220
   Asp Glu Leu Pro Gln Glu Thr Arg Phe Leu Gln Leu Phe Asn Arg Arg
   225 230 235 240
   His Phe Pro Phe Val Leu Phe Val Ala Ala Ile Trp Thr Cys Gln Val
   245 250 255
   Ile Pro Met Phe Ala Ile Tyr Thr Phe Gly Pro Gln Ile Val Gly Leu
   260 265 270
   Leu Gly Trp Glu Gln Gly Arg Asn Ala Ala Leu Gly Asn Val Val Ile
   275 280 285
   Ser Leu Phe Phe Met Leu Gly Cys Ile Pro Ala Met Phe Trp Leu Asn
   290 295 300
   Ser Ile Gly Arg Arg Pro Leu Leu Ile Gly Ser Phe Ala Met Met Thr
   305 310 315 320
   Ile Ala Leu Ala Leu Leu Gly Leu Val Ser Asn Leu Gly Ile Ile Leu
   325 330 335
   Val Val Val Ala Phe Ala Val Tyr Ala Phe Phe Ser Gly Gly Pro Gly
   340 345 350
   Ile Leu Gln Trp Leu Tyr Pro Asn Glu Leu Phe Pro Thr Asp Ile Arg
           355                 360                 365
   Ala Ser Ala Val Gly Val Ile Met Ser Leu Ser Arg Ile Gly Thr Ile
       370                 375                 380
   Val Ser Thr Trp Ala Leu Pro Ile Phe Ile Thr Arg Tyr Gly Ile Asn
   385                 390                 395                 400
   Asn Val Met Leu Ile Gly Ala Leu Ile Ser Leu Val Gly Leu Gly Val
                   405                 410                 415
   Ser Val Met Phe Ala Pro Glu Thr Arg Gly Leu Thr Leu Thr Gln Thr
               420                 425                 430
   Gly Asn Met Thr Leu Arg Gly Thr Pro Ser Asp Asn Pro Arg
           435                 440                 445